(19) Europäisches Patentamt

European Patent Office

Office européen des brevets

(11) **EP 1 416 443 A1**

(12) **EUROPEAN PATENT APPLICATION**

(43) Date of publication:
**06.05.2004 Bulletin 2004/19**

(51) Int Cl.⁷: **G06T 17/00**, G01S 15/89,
G06T 15/00, A61B 8/00

(21) Application number: **03254092.4**

(22) Date of filing: **27.06.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **28.10.2002 JP 2002312142**

(71) Applicant: **Kabushiki Kaisha Toshiba**
**Tokyo 105-8001 (JP)**

(72) Inventor: **Hashimoto, Keisuke**
**1-1 Shibaura 1-chome Minato-ku Tokyo (JP)**

(74) Representative: **Midgley, Jonathan Lee**
**Marks & Clerk**
**57-60 Lincoln's Inn Fields**
**GB-London WC2A 3LS (GB)**

(54) **Image processing apparatus and ultrasound diagnosis apparatus**

(57)     A volume data acquired from a subject, which is allocated in points constituting three-dimensional space and forming a data set representing a physical property of the subject, is recorded in a recording means (39). A characteristic quantity computed from values of the physical property held by the volume data at each point is extracted by a characteristic quantity extracting means (33) and a three-dimensional image is generated by providing opacity to the characteristic quantity by three-dimensional image generating means (37). The volume data is voxel volume data or polar-coordinate ultrasound volume data. In this way, the internal structure of parenchymatous organs, especially internal blood vessels and cavitary structures, can be displayed three dimensionally.

FIG. 1

**Description**

BACKGROUND OF THE INVENTION

1. Field of the Invention

The present invention relates to an image processing apparatus and an ultrasound diagnosis apparatus for imaging three-dimensional volumes for representing physical properties of a subject.

2. Description of the Related Art

In recent years, in venues of medical acts such as diagnosis and treatment, images created on medical image diagnosing apparatus such as ultrasound diagnosis apparatuses, X-ray CT apparatus, X-ray diagnosis apparatus, magnetic resonance imaging (MRI) apparatus, nuclear medicine diagnosis apparatus (gamma cameras), are being displayed as three-dimensional images for performing diagnosis or treatment. In the field of such three-dimensional image diagnosis, oftentimes, images are acquired by volume for example, a three-dimensional image is displayed by volume rendering (hereafter may be referred to as "VR"), and a physician finding disorders or the like by reading the three-dimensional image.

Volume rendering involves layering slice images obtained by an ultrasound diagnosis apparatus or the like for example, then creating a volume model (voxel space) having a three-dimensional structure wherein the values of each of a plurality of the slice images are packed into squares called voxels, a visual line direction is determined regarding this volume model and voxel tracking (ray tracing) is performed from an arbitrary viewpoint, thereby obtaining the brightness (voxel value) at the voxels, and on pixels on a projection plane, projecting image information based on this brightness, thus extracting the liver and the like three-dimensionally to obtain a three-dimensional image.

Unlike surface rendering, volume rendering can easily display a three-dimensional structure even in the event that clear boundary lines cannot be extracted, and unlike rendering methods such as MIP (maximum intensity projection), images containing even more accurate position information can be displayed.

For example, with three-dimensional image processing using an ultrasound diagnosis apparatus, ultrasound vector data collected by manually or mechanically scanning with an ultrasound probe is temporarily converted into voxel volume data made up of voxels on orthogonal X-Y-Z axes by a digital scan converter. The voxel volume is subjected to volume rendering at a three-dimensional rendering unit, and a three-dimensionally-rendered image is displayed on a display unit such as a CRT. This is described in Japanese Unexamined Patent Application Publication No. 2002-224109, paragraphs [21] through [53], for example.

Further, ultrasound diagnosis apparatuses display tomographic images of tissue through non-invasive inspection, enabling real-time display of the heart beating or a fetus moving with the simple operations of just bringing an ultrasound probe into contact with the surface of the body, and can perform blood flow imaging by the ultrasound Doppler method, as an example of the unique features of ultrasound diagnosis apparatuses.

However, in the event of attempting three-dimensional image display, volume rendering image display for example, based on images collected by the ultrasound diagnosis apparatus, since cavities with no blood flow such as a gall bladder, and tubular structured tissues do not yield Doppler signals, there is the problem that when three-dimensionally visualizing parenchymatous organs, such as a liver, the internal structure of the organ is hardly seen, and internal blood vessels and cavitary structures cannot be displayed.

Even if a parameter called opacity (for how much the inside can be seen through) is set and the luminance of the values of the original image is adjusted corresponding to opacity (or transparency), the boundary faces of internal structures could not be clearly displayed.

In order to solve this problem, in the event of spatially comprehending a B/W tissue tomography image for example, the three-dimensional structure is comprehended by performing clipping operations such as box clipping (setting a box-shaped visible region, so only inside this region is the object of display), cross-section positioning operation of an MPR (multi planar reconstruction) image, and so forth.

Or, color Doppler may be used to combine blood flow information and a B/W tissue tomography image for display.

However, there is the need to perform fine settings using a mouse while rotating the volume in order to carry out clipping or MPR image positioning, so in the event of displaying a three-dimensional image in real-time and observing the changes therein, such as blood flow, a technician must hold the ultrasound probe for sequentially taking in the three-dimensional volumes and simultaneously perform complicated operations for volume rendering, such as clipping processing and the like, so this arrangement is unrealistic from the viewpoint of operability.

Besides, there is the problem that the internal structure cannot be comprehended unless the cross-section is referred to by clipping and the like with regard to the volume rendering image, and the task of cutting the cross-section with a mouse or the like is very troublesome.

Particularly, cavities with no blood flow such as the gall bladder or tissue having tubular structures do not yield Doppler signals, so cavitary structures with no blood flow have not been able to be displayed, even using the color Doppler method. While a method for obtaining Doppler signals by injecting an ultrasound contrast agent might be conceived, this in itself has problems of increased invasiveness, inspection becoming less handy, and so forth.

In order to achieve the object, as one aspect of the invention, there is provided an image processing apparatus (2, 102, 212) comprising: recording means (39) for recording volume data acquired from a subject, which is allocated in three-dimensional space, and forms a data set representing a physical property of the subject; characteristic quantity extracting means (33) for extracting a characteristic quantity computed from values of the physical property held by each volume data; and three-dimensional image generating means (36, 37) for providing opacity to the characteristic quantity, and for generating a volume rendering image using the opacity.

Preferably, the characteristic quantity is boundary information representing a boundary face between different objects existing inside the volume data. In this case, the three-dimensional image generating means may heighten the opacity of the boundary face, and lower the opacity of a rest so as to generate a volume rendering image with the boundary face enhanced. Besides, the characteristic quantity extracting means may compute one of a normal vector perpendicular to the boundary face and information regarding the vector length (S4), which is determined from the difference between an intensity of volume data of interest and an intensity of nearby volume data. Further, the three-dimensional image generating means may generate a volume rendering image based on one of the normal vector and the information regarding the vector length.

It is preferred that the characteristic quantity extracting means computes a gradient vector necessary for shading processing (S609), and the three-dimensional image generating means generates a volume rendering image using one of the gradient vector and a value of its intermediate product made in the process of its computation.

It is also preferred that the characteristic quantity extracting means is configured with a high-pass filter processing the volume data of the interest, or comprises three Sobel filters (335a, 335b, 335c) mutually independently processing the volume data in three directions set to identify a position of the volume data in the three-dimensional space. Further, a smoothing means (31) for performing smoothing processing may added before performing characteristic quantity extraction processing. In this case, the smoothing means may be one of a weighted averaging unit and a median filtering unit (331).

Preferably, one of the characteristic quantity extracting means and the three-dimensional image generating means performs processing (S3) in increments of slices parallel to the two directions out of the three directions, and the closest to perpendicular to a projection direction.

Still preferably, the image processing apparatus further comprises a display means (38) for displaying an animated image by sequentially processing a plurality of volume data recorded in the recording means. In this case, the display means may sequentially performs processing consecutive volume data in real time acquired with two-dimensional array probe which can scan a three-dimensional space in order to display an animated image.

It is preferred that the three-dimensional image generating means generates a plurality of tomographic images cut in different directions. In this case, the three-dimensional image generating means may generate at least one of the plurality of tomographic images cut in different directions and a volume rendering image based on a value of the volume data, in concurrence with generating a volume rendering image, and the display means may displays them (402, 404) simultaneously.

It as also preferred that the characteristic quantity extracting means performs characteristic quantity extraction processing only on a certain type of volume data among a plurality of types of volume data with different physical properties, and the three-dimensional image generating means generates a three-dimensional image by superimposing three-dimensional distribution information acquired from the volume data processed in the characteristic quantity extraction means on three-dimensional distribution information acquired from the remaining unprocessed volume data. In this case, the characteristic quantity extraction means is configured wherein a selection condition of a type of volume data to be processed may be changeable so that the characteristic quantity extraction processing is performed on a different type of volume data.

As another aspect of the invention, there is provided that an ultrasound diagnosis apparatus (1, 100, 210) comprising: ultrasound transmission/reception means (14, 22) for transmitting ultrasound waves to a subject and receiving reflected waves from the subject so as to outputting volume data acquired from a subject, which is allocated in three-dimensional space, and forms a data set representing a physical property of the subject as signals from the subject; first ultrasound information generating means (27) for acquiring and outputting first three-dimensional distribution information about a tissue structure of the subject; second ultrasound information generating means (28) for acquiring and outputting second three-dimensional distribution information about property of a moving object of the subject; recording means (39) for recording volume data acquired by the ultrasound transmission/reception means; characteristic quantity extracting means (33) for extracting a characteristic quantity computed from values of the physical property held by each volume data; and three-dimensional image generating means (37) for providing opacity to the characteristic quantity, and for generating a volume rendering image using the opacity.

Preferably, the volume data is acquired by the ultrasound transmission/reception means during scanning a section of the subject with the use of one of a two-dimensional array probe (12) and swing movement of a sector probe, and represented by polar coordinates, whose origin is set at an irradiating point of ultrasound beam, using two angles in mutually orthogonal directions. Or the volume data is acquired by the ultrasound transmission/reception means during scanning a section of the subject by rotating a ultrasound probe around its axis so as to rotate a plurality of volume data of interest disposed in a two-dimensional plane around the axis in the opposite way. Or again, the volume data is acquired by the ultrasound transmission/reception means during scanning a section of the subject by shifting a ultrasound probe in parallel along a perpendicular direction to the section so as to shift a plurality of volume data of interest in parallel to the opposite direction.

BRIEF DESCRIPTION OF THE DRAWINGS

FIG. 1 is a functional block diagram illustrating an example of the overall schematic configuration of an ultrasound diagnosis apparatus according to the first embodiment of the present invention;
FIG. 2 is a functional block diagram illustrating details of a face extraction filter processing unit of the ultrasound diagnosis apparatus shown in FIG. 1;
FIGS. 3A through 3C are explanatory diagrams for describing the overview of processing at the face extraction filter processing unit, in which FIG. 3A illustrates an array of the eight samples (voxels) near the sample of interest in the X-direction in the image, FIGS.3B and 3C illustrate those in the Y-direction and Z-direction respectively;
FIGS. 4A and 4B are explanatory diagrams for describing the overview of processing at the smoothing filer processing unit, in which FIG. 4A illustrates volume data including the sample (voxel), and FIG. 5B illustrates the six nearby samples;
FIG. 5 is a flowchart describing a specific example of processing of a median filter;
FIGS. 6A and 6B illustrate some examples of a volume scan, in which, FIG. 6A illustrates shifting a ultrasound probe in parallel along a perpendicular direction to the section, and FIG. 6B illustrates rotating a ultrasound probe around its axis;
FIGS. 7A and 7B are explanatory diagrams for comparing a three-dimensional image generated by the ultrasound diagnosis apparatus according to the present invention with a three-dimensional image generated by a conventional ultrasound diagnosis apparatus, in which FIG. 7A illustrates a liver displayed on the display unit according to a normal mode and FIG. 7B illustrates a liver displayed on the display unit according to the present invention;
FIG. 8 is a functional block diagram illustrating the details of another example of a face extraction filter processing unit according to the second embodiment of the ultrasound diagnosis apparatus according to the present invention;
FIG. 9 is a functional block diagram illustrating an example of the overall schematic configuration of the ultrasound diagnosis apparatus according to the third embodiment of the present invention;
FIGS. 10A through 10C are explanatory diagrams for describing the geometric shape of ultrasound volume data collected by an ultrasound probe, in which FIG. 10A illustrates a geometric shape of a volume, FIG. 10B illustrates the angle $\theta$ between the projected ultrasound beam on the X-Y plane and the Y axis, and FIG. 10C illustrates the angle $\psi$ between the projected ultrasound beam on the Y-Z plane and the Y axis;
FIG. 11 is a functional block diagram illustrating a detailed configuration of a slice processing unit of the ultrasound diagnosis apparatus shown in FIG. 9;

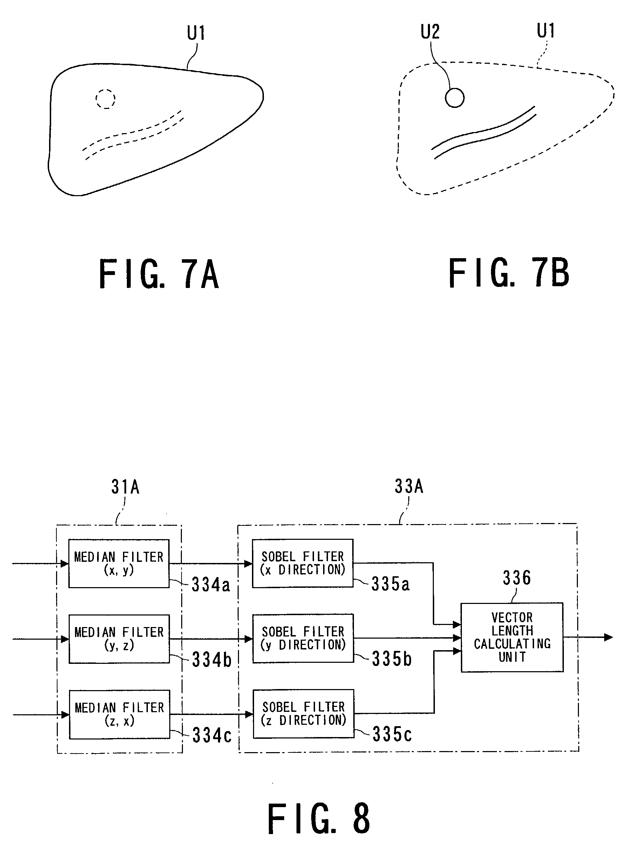

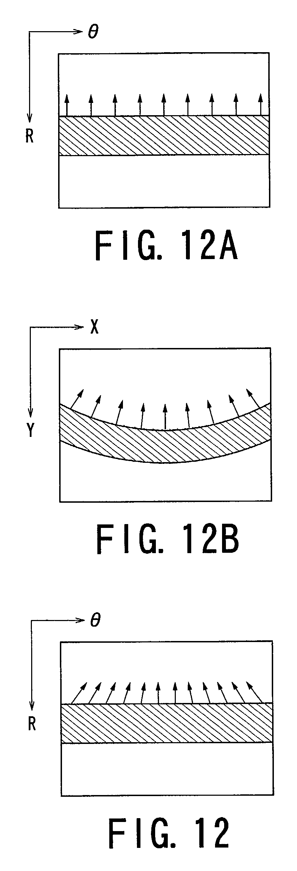

FIGS. 12A through 12C are conceptual diagrams for describing conversion processing for converting normal vectors on a polar-coordinate into those on an orthogonal coordinates, which is performed by a shading vector computation unit of the ultrasound diagnosis apparatus shown in FIG. 8, in which Fid. 12A illustrates ultrasound slice data on polar coordinates that are input to the shading vector computation unit, FIG. 12B illustrates the ultrasound slice data on a polar coordinate system shown in FIG. 12A that has been represented by orthogonal coordinates and FIG. 12C is a conceptual diagram of the output data of the shading vector computation unit;
FIG. 13 is a functional block diagram illustrating the detailed configuration of a shading vector computation unit of the ultrasound diagnosis apparatus shown in FIG. 9;
FIG. 14 is a functional block diagram illustrating the detailed configuration of a slice rendering unit of the ultrasound diagnosis apparatus shown in FIG. 9;
P8 L13
FIGS. 15A through 15C are explanatory diagrams for describing the concept of image generation processing in the event that the visual line direction is set in the $\varphi$ axis direction, in which FIG. 15A illustrates an ultrasound slice data group being generated from the obtained ultrasound volume data, FIG. 15B illustrates the ultrasound slice data being geometrically converted and superimposed by rendering processing and FIG. 15C illustrates component shapes geometry corresponding to the slices;

FIGS. 16A through 16C are explanatory diagrams for describing the concept of image generation processing in the event that the visual line direction is set in the R axis direction in which FIG. 16A illustrates an ultrasound slice data group being generated from the obtained ultrasound volume data, FIG. 16B illustrates the ultrasound slice data being geometrically converted and superimposed by rendering processing and FIG. 16C illustrates component shapes geometry corresponding to the slices;

FIG. 17 is a flowchart illustrating an example of ultrasound image collecting and generating processing procedures with an ultrasound diagnosis apparatus according to the third embodiment of the present invention;

FIG. 18 is a flowchart describing an example of slice processing performed by the slice processing unit of the ultrasound diagnosis apparatus shown in FIG. 9;

FIGS. 19A through 19C are explanatory diagrams for describing the relation between the visual line direction and slice face, in which FIG. 19A illustrates a R-ψ slice face with the same θ, FIG. 19B illustrates a R-θ slice face with the same ψ, and FIG. 19C illustrates a θ-ψ slice face with the same R;

FIG. 20 is a flowchart describing an example of the processing procedures executed at the slice rendering unit of the ultrasound diagnosis apparatus shown in FIG. 9;

FIG. 21 is an explanatory diagram describing the correlation between R-φ slice face and R-θ slice face ultrasound slice data, and slice geometric information;

FIG. 22 is an explanatory diagram describing the correlation between φ-θ slice face ultrasound slice data and slice geometric information;

FIG. 23 is a functional block diagram illustrating an example of the overall schematic configuration of the ultrasound diagnosis apparatus according to the fourth embodiment of the present invention;

FIG. 24 is a functional block diagram illustrating a detailed configuration of the shading vector computation unit of the ultrasound diagnosis apparatus shown in FIG. 23;

FIG. 25 is a flowchart illustrating an example of ultrasound image collecting and generating processing procedures with the ultrasound diagnosis apparatus shown in FIG. 22;

FIG. 26 is a flowchart illustrating an example of face extracting processing procedures with the ultrasound diagnosis apparatus shown in FIG. 23;

FIG. 27 is a functional block diagram illustrating an example of the overall schematic configuration of the ultrasound diagnosis apparatus according to the fifth embodiment of the present invention;

FIG. 28 is an explanatory diagram describing an example of the display format displayed on the display unit; and

FIG. 29 is a functional block diagram illustrating an example of the overall schematic configuration of the ultrasound diagnosis apparatus according to seventh embodiment of the present invention.

DESCRIPTION OF THE PREFERRED EMBODIMENTS

The following is a specific description of an example of preferred embodiments of the present invention, with reference to the drawings. In the following, an embodiment wherein samples of voxel volume data (voxels) are subjected to face extraction filtering will be described in the "first embodiment", and an embodiment wherein samples of polar-coordinate ultrasound volume data are subjected to face extraction filtering will be described in the "third embodiment". Other embodiments are all various modifications. The description will now begin with the first embodiment.

First Embodiment

First, with the first embodiment, face extraction processing (high band enhancing filtering processing), which is a feature of the present embodiment, is performed on an equant voxel volume, generating a volume with enhanced face component, and volume rendering processing is performed regarding each sample value, thereby displaying a volume rendering image with enhanced face components.

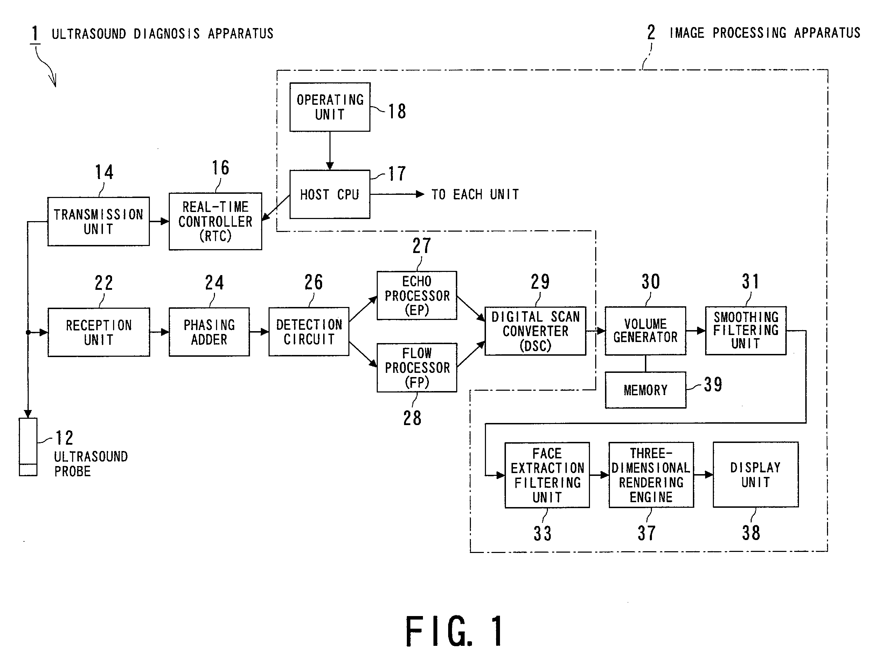

Before describing such features, the overall schematic configuration of the ultrasound diagnosis apparatus which is the basis thereof will be described with reference to FIG. 1. FIG. 1 is a block diagram illustrating an example of the configuration of the ultrasound diagnosis apparatus according to the present embodiment.

(Configuration of ultrasound diagnosis apparatus)

As shown in FIG. 1, the ultrasound diagnosis apparatus 1 according to the present embodiment comprises an ultrasound probe 12 for handling transmission and reception of ultrasound signals between the device and a subject, a transmission unit 14 for driving the ultrasound probe 12, a reception unit 22 for processing the reception signals from the ultrasound probe 12, a phasing adder 24, a detection circuit 26, an echo processor (EP) 27 which is a B/W luminance signal processing unit, a flow processor (FP) 28 which is a blood flow detecting/processing unit, a digital scan converter (DSC) 29, a real-time controller (RTC) 16 which is a transmission/reception control circuit, a host CPU 17 which is a control unit, a volume generator 30, a smoothing filtering unit 31, a face extraction filtering unit 33, a three-dimensional rendering engine 37, a display unit 38 for displaying three-dimensional images and the like, a memory 39, an operating unit 18 capable of receiving input of instruction information from an operator, and so forth. Note that reference numeral 2 denotes the configuration of the image processing apparatus.

The ultrasound probe 12 is a probe for transmitting photographing ultrasound waves into the subject (patient) and receiving the reflected waves from the subject, and is made of piezoelectric transducers and so forth. The piezoelectric transducers are cut in a direction perpendicular to the scanning direction, and make up a plurality of channels. Manually or mechanically scanning with the ultrasound probe 12 in a direction perpendicular or generally perpendicular to the scan cross-section collects three-dimensional ultrasound volumes. The manual or mechanical scanning position is detected by an unshown magnetic sensor or encoder, and the scanning position information is input to the real-time controller (RTC) 16 where header information is added and sent to the volume generator 30 along with the ultrasound wave data.

A real-time controller (RTC) 16 performs timing control for transmission/reception of ultrasound signals, based on scan control parameters input from the host CPU 17. Included in the control parameters are ultrasound collection mode such as B/W or color Doppler scan, scan region, raster density, repetition cycle of ultrasound data collection, and so forth. The real-time controller (RTC) 16 operates a timer based on repetition cycle information of the ultrasound data collection, and generates ultrasound transmission reference signals based on the cyclically generated timer output.

The real-time controller (RTC) 16 also generates information necessary for beam processing, such as a beam type for distinguishing whether the ultrasound beam is B/W data or color Doppler data, data collection distance, and so forth, as header information. The generated header information is added to the data in the later-described reception/transmission unit 22, and is transmitted to the units for performing the subsequent processing with the data. The units downstream determine the contents of beam processing, beam type identification and beam processing and parameters based on the received header information, and following execution of necessary processing, further combines the header information and ultrasound beam data which is transferred to the units downstream.

Though not shown in the drawings, the transmission unit 14 has a basic pulse generator, a delay circuit, and a high-voltage pulse generating circuit (pulser circuit). The transmission unit 14 generates transmission pulse generating signals with the basic pulse generator using the ultrasound transmission/reception reference signals input from the real-time controller (RTC) 16 as a reference, adds delay time for forming desired ultrasound beams with the delay circuit channel by channel, amplifies transmission pulse generating signals with the pulser circuit, and applies them to the piezoelectric transducers making up each channel of the ultrasound probe 12.

Though not shown in the drawings, the reception unit 22 has a preamplifier, an A/D converter, and a reception delay circuit. The reception unit 22 receives ultrasound reflection pulses from the subject channel by channel in the ultrasound probe 12 under control of the real-time controller 16, which are converted into digital signals at the A/D converter following amplification of the amplitude thereof by the preamplifier.

Thus, reception signals are obtained by generating pulsed ultrasound waves which are sent to transducers of the ultrasound probe 12, and receiving the echo signals scattered in the tissue of the subject with the ultrasound probe 12 again.

The output from the reception unit 22 is subjected to delay processing necessary for determining reception directivity at the phasing adder 24 and then addition processing to form a plurality of ultrasound beams for each raster, the ultrasound beam data is subjected to quadrature phase detection processing in the detection circuit 26, and is sent to the echo processor (EP) 27 or the flow processor (FP) 28 according to the imaging mode.

The phasing adder 24 performs addition processing for the signals of the reception channels input from the reception unit 22, taking into account delay time necessary for determining the reception directivity using an unshown digital delay phasing adder, and outputs obtained RF (Radio Frequency) ultrasound signals thereto. The RF ultrasound signal corresponds to the ultrasound beam of each raster formed by delay addition processing. Forming a plurality of ultrasound beams simultaneously at the phasing adder 24 enables so-called parallel simultaneous reception, so that the scanning time of the ultrasound volume can be reduced.

The detection circuit 26 subjects the ultrasound beam data formed by the delay addition processing at the phasing adder 24 to quadrature phase detection processing, and sends the processed signals to the echo processor (EP) 27 or the flow processor (FP) 28 according to the imaging mode.

The echo processor (EP) 27 is a unit for performing signal processing necessary for generating three-dimensional B/W tissue image data indicating the tissue structure information involved in the reception signals reflected from the body tissue. Specifically, the echo processor (EP) 27 forms pictures of the intensity of ultrasound signals reflected at the tissue by envelope detection processing, and performs high-cut filtering suitable for generating image data corresponding to the tissue structure.

The flow processor (FP) 28 making up a blood flow signal detection/processing unit is a unit for performing signal processing necessary for forming pictures of the movement such as blood flow and the like, and specifically, parameters such as velocity, power, dispersion and so forth are calculated with the color Doppler method. The output of the echo processor (EP) 27 or the flow processor (FP) 28 is data for each sample position along with the direction of the ultrasound beam (hereafter referred to as "ultrasound sample data"), and a three-dimensional volume configured of the ultrasound sample data will be referred to as ultrasound volume data (previously "ultrasound vector data set").

The digital scan converter (DSC) 29 is for converting a train along each raster scanned by ultrasound scanning into a train along each raster in a common video format such as television format, wherein the data input from the echo processor (EP) 27 is used to generate B/W tissue image data, and the data input from the flow processor (FP) 28 is used to generate color blood flow image data, based on geometrical information of each ultrasound raster, and both are weighted for example and added to generate display image data. Interpolation using commonly-known anti-aliasing is performed for data where aliasing occurs such as in the blood flow velocity, thereby generating a two-dimensional image.

The volume generator 30 converts the plurality of tomography images input from the digital scan converter (DSC) 29 into volumes configured of equant voxels, based on the scan cross-section position information. Here, linear interpolation processing (Tri-Linear interpolation processing) using the eight ultrasound samples surrounding the voxel of interest is employed for the interpolation processing. With regard to data wherein aliasing, as typified by blood flow velocity occurs, Tri-Linear interpolation processing including the anti-aliasing processing is performed.

The image memory 39 is coupled with the Volume generator 30, and includes a memory device and a writing/reading controller for storing therein data handled by the Volume generator 30 (i.e., either one type of data conformable to ultrasound scanning or standard television scanning). Echo data stored in the memory device can be read by the unit of frame during real-time imaging or after such imaging in response to an operator's command. The read data are sent via the volume generator 30 and so forth to the display unit 38 to be displayed thereon.

The smoothing filtering unit 31 performs smoothing processing on the three-dimensional volume generated by the volume generator 30, and removes noise such as speckle noise.

The face extraction filtering unit 33 performs low-cut filtering on the three-dimensional volume of the volume generator 30, so as to generate a three-dimensional volume wherein the face component is enhanced.

The three dimensional rendering engine 37 receives the voxel volume which the volume generator 30 has generated and has been subjected to smoothing and face extraction processing, and generates a three-dimensional rendering image based on image generating parameters set in the CPU 17, including volume rendering, surface rendering, rendering mode such as MPR, as well as visual line direction, opacity, coloring method, and so forth. Note that while various techniques are being proposed for algorithms for generating three-dimensional images, a commonly-known one is ray tracing.

The display unit 38 is composed of a CRT (cathode ray tube) or LCD (liquid crystal display) monitor, and is used for displaying two-dimensional ultrasound images such as the B/W tissue images or color blood flow images or the like generated by the digital scan converter (DSC) 29, and diagnosis of the subject by the user. The display unit 38 also displays a three-dimensional rendering image generated by the three-dimensional rendering engine 37 either independently, or along with the two-dimensional ultrasound images generated by the digital scan converter (DSC) 29.

Particularly, the display unit 38 is arranged so as to be capable of displaying three-dimensional images subjected to face enhancement (first three-dimensional images), three-dimensional images not subjected to face enhancement (second three-dimensional images), MPR images according to one or both of them, and so forth. These can be switched over as appropriate by a display control unit contained in the host CPU 17, according to operating instructions from the operating unit 18.

Thus, an image representing the tissue shape of the subject is displayed on the display unit 38, and the user can obtain three-dimensional information from the ultrasound image displayed thereupon, and accordingly can easily obtain a general understanding of whether or not there is a disorder, and if so, the size and so forth of the affected area.

The operating unit 18 has devices for inputting predetermined instructions, such as a mouse, buttons, keyboard, trackball, operating panel, and so forth. These operating devices are used for the operator to input or set patient information, device conditions, and so forth, and also are used for inputting necessary transmission/reception conditions, display format selection information, specifying MPR cross-section on a three-dimensional image, setting rotations and opacity of the three-dimensional image, and so forth.

For example, conditions relating to scanning and displaying are input by operating switches disposed on the operating panel, or by using the mouse or the like to select one from a menu within a window displayed on the display unit 38 making up the image display unit composed of a CRT or the like. Also, rotation operations with regard to the ultrasound volume data, display window level and opacity/color settings, and so forth, are performed by moving the mouse vertically and horizontally.

The host CPU 17 is a control means serving as the control center of the entire apparatus to control the components, and has functions of an information processing device with memory (i.e., a computer) so as to control the actions of the ultrasound diagnosis apparatus itself following procedures programmed beforehand. The CPU 17 controls the transmission unit 14 and the reception unit 22 connected to the ultrasound probe 12, the phasing adder 24, the detection circuit 26, the echo processor (EP) 27 for obtaining images of the subject, the flow processor (FP) 28 for obtaining blood flow images, the volume generator 30 for generating volumes, the digital scan converter (DSC) 29, the smoothing filter processing unit 31, the face extraction filter processing unit 33, the three-dimensional rendering engine 37, the display unit 38, and so forth.

The control actions include processing regarding the diagnosis mode, transmission/reception conditions, display format such as three-dimensional image display or MPR images or the like, which the operator commands via the operating unit 18, and also includes transmission control (transmission timing, transmission delay, etc.) regarding the transmission unit 14, reception control regarding the reception unit 22 (reception delay, etc.), commands for generating three-dimensional images from the three-dimensional rendering engine 37, and further, calling up and executing necessary programs and data in the face extraction and so forth regarding three-dimensional images according to the present invention, instructing face extraction processing at the face extraction filtering unit 33, prompting executing of MPR processing and the like, and overall control of software modules.

The host CPU 17 interprets the conditions relating to scanning or displaying input via the operating unit 18 by the user, and controls the entire apparatus by setting parameters necessary for such control. Upon completion of setting the parameters for the entire apparatus, the host CPU 17 instructs the real-time controller (RTC) 16 to start transmission/reception of ultrasound signals.

The host CPU 17 successively judges the operation inputs successively made by the user via the operating unit 18 as to the three-dimensional images, such as rotation operations for the volume, and performs control regarding display of three-dimensional images by setting the necessary parameters to the three-dimensional rendering engine 37 and so forth.

Besides, the two-dimensional ultrasound images and the three-dimensional images and the like are stored in the memory 39, and can be called up by the operator following diagnosis for example. Also, the memory 39 not only saves the diagnosis images, but also stores various types of software programs for performing the aforementioned face extraction filtering and for programs for performing smoothing to remove speckle noise or the like.

Further, the host CPU 17 reads in outputs signals or image luminance signals immediately after the reception unit 22, and displays these on the display unit 38 via the digital scan converter (DSC) 29, or saves the signals in the memory 39 as an image file, or transfers the signals to an external information processing device (PC), printer, external storage medium, diagnosis database, electronic medical record system, and so forth, via another interface.

(Overall operations of ultrasound diagnosis apparatus)

The ultrasound diagnosis apparatus 1 having such a configuration operates generally as described below. That is, upon diagnosis being commanded, ultrasound waves transmitted from the transmission unit 14 to the body as the subject via the ultrasound probe 12 is received at the reception unit 22 via the ultrasound probe 12 again as reflected signals from the body. The echo signals subjected to phasing addition passing through the reception unit 22, and subjected to logarithmic amplification and envelope detection being output as luminance information with amplitude information, then input to the digital scan converter (DSC) 29 as an image. This yields a normal two-dimensional tomography image.

The output of the reception unit 22 is subjected to delay processing at the phasing adder 24 necessary for determining reception directivity, following which addition processing is performed to form a plurality of ultrasound beams for each raster, and quadrature phase detection processing is performed with regard to the ultrasound beam data at the detection circuit 26 (the implementation up to this point configures the ultrasound transmission/reception means according to the present invention), which is sent to the echo processor (EP) 27 or the flow processor (FP) 28 according to the imaging mode.

The echo processor (EP) 27 forms pictures of the intensity of ultrasound signals reflected at the body tissue by envelope detection processing, and performs high-cut filtering and the like suitable for generating image data (B/W tissue images) corresponding to the tissue structure. Here, the echo signals are subjected to various types of filtering, logarithmic amplification, envelope detection processing, and so forth, and become data wherein the signal intensity is represented as luminance.

On the other hand, the flow processor (FP) 28 performs signals processing necessary for forming pictures of the movement of moving objects such as blood flow or the like, i.e., the intensity of ultrasound signals reflected on the moving objects, by envelope detection processing, and parameters such as velocity, power, dispersion, and so forth are calculated using intensity reflected on the moving objects by the color Doppler method, for example (the above EP 27 and FP 28 are the ultrasound information generating means in the present invention). Velocity information is also obtained from the echo signals by frequency analysis, and the results of the analysis are sent to the digital scan converter (DSC) 29.

The digital scan converter (DSC) 29 then generates a B/W tissue image from the data input from the echo processor (EP) 27 based on the geometric information from each ultrasound raster, and also generates a color blood flow image from the data input from the flow processor (FP) 28, and weights and adds the both to generate display image data. In addition, interpolation using commonly-known anti-aliasing is performed for data where aliasing occurs such as in the blood flow velocity, thereby generating a two-dimensional image.

The image data sent to the digital scan converter (DSC) 29 is subjected to post-processing such as smoothing, and then is subjected to scan conversion into a video format image data. This image data is further sent to the display unit 38 in real-time. AT this time, necessary graphic data is superimposed and displayed on the display unit 38.

The image data before and after scan conversion is stored in the memory 39, and can be read out and reused by the operator, i.e., displayed or the like. At this time, the images read out from the memory 39 can be viewed under display control such as slow-motion playback, frame-by-frame playback, freeze-frame, and so forth.

Now, upon the operator making transition to the mode for three-dimensional display, a three-dimensional image is displayed based on the image data stored in the memory 39 on the display unit 38.

(Three-dimensional display)

In order to perform three-dimensional image display, the volume generator 30 converts the input a plurality of tomography images into volumes configured of equant voxels, based on the scan cross-section position information.

The smoothing filtering unit 31 performs smoothing on the three-dimensional volume generated by the volume generator 30, so as to remove noise such as speckle noise or the like, and further, the face extraction filtering unit 33 performs low-cut filtering on the three-dimensional volume, so as to generate a three-dimensional volume wherein the face component is enhanced.

The three-dimensional rendering engine 37 receives the voxel volume which the volume generator 30 has generated and which has been subjected to smoothing and face extraction processing, and generates a three-dimensional rendering image based on image generating parameters set in the CPU 17, including volume rendering, surface rendering, rendering mode such as MPR, and so forth, as well as visual line direction, opacity, coloring method, and so forth.

In this manner, various formats of images, such as image data or graphic data being transmitted, normal-mode three-dimensional images commanded by the host CPU 17, images from the face extraction filtering unit 33 and so forth, are input to the display unit 38 appropriately.

Thus, the display unit 38 displays a two-dimensional ultrasound image such as a B/W tissue image or color blood flow image of a subject, or a three-dimensional rendering image, the MPR image thereof, and so forth, either independently, or along with the two-dimensional ultrasound images, as necessary.

At this time, in the three-dimensional rendering image, face component or the outline of the three-dimensional internal structures of parenchymatous organs, such as blood vessels or tumors or the like within the gall bladder or liver for example, have been subjected to enhancement by the filtering at the face extraction filtering unit 33, so that the shapes and the like of the blood vessels, cavities, and tumors, are clearly displayed.

In addition, an arrangement may be made for displaying the two-dimensional ultrasound image or the three-dimensional rendering image, wherein graphic data and the like of information regarding various setting parameters and so forth is generated by an unshown data generating unit, and the image is synthesized with the use of the memory 39 and the like, thereby outputting the synthesized image to the display unit 38.

The finalized image data thus generated is displayed on the display unit 38, and in the event that the "3D mode" for displaying a three-dimensional image has been selected, the display unit 38 normally displays a three-dimensional image of the liver for example, by volume rendering, and displays a face-enhanced image wherein the internal structures within the liver for example, such as a tumor or the like, has been face-enhanced, by the user selecting a certain display operating portion. Note that with the two-dimensional ultrasound image, a desired portion or data is subjected to coloring thereupon if necessary.

An even more detailed configuration for performing face extraction filtering processing and the like with the above configuration will be described below in detail.

(Features of the present invention: configuration for performing face extraction)

With the present embodiment, the following configuration is assumed to perform face extraction on three-dimensional volume data. A case of performing face extraction processing on a voxel-shaped volume will be described with the present embodiment.

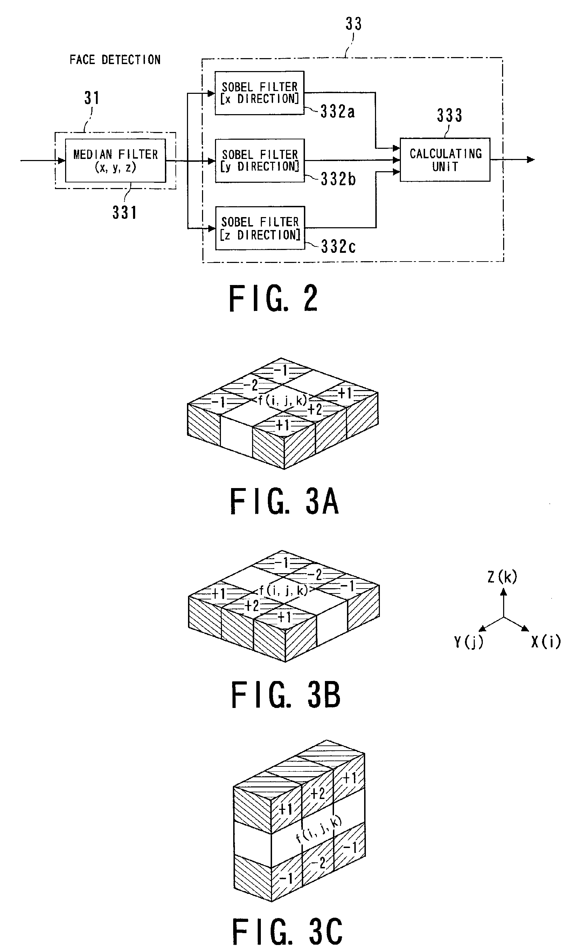

As shown in FIG. 2, the ultrasound diagnosis apparatus according to the present embodiment comprises the smoothing filtering unit 31 for removing speckle noise and the like from three-dimensional volume data generated at the volume generator 30, and the face extraction filtering unit 33 for extracting or enhancing the outline of a tumor in a liver or the like (the boundary between the surface of a tumor and a full portion in a liver) with regard to the three-dimensional volume data, and performing face extraction.

That is, with the present apparatus, smoothing is performed with a median filter in the smoothing filtering unit 31, following which the magnitude of the face component is detected by Sobel-type 3 by 3 high-pass filters 332a, 332b, 332c of the face extraction filtering unit 33. These are each executed in increments of volumes.

To define a few terms, the term "face extraction filtering unit" in the present embodiment corresponds to the "characteristic quantity extraction means" according to the preset invention, the term "smoothing filtering unit" in the present embodiment corresponds to the "smoothing means" according to the preset invention, the term "three-dimensional rendering engine" in the present embodiment corresponds to the "three-dimensional image generating means" according to the preset invention, and further, the "memory" in the present embodiment may comprise the "recording means" according to the preset invention.

(Face extraction filter)

As shown in FIG. 2, the face extraction filtering unit 33 has functions of extracting the face components of three-dimensional volume data, and is configured including an X-directional filtering unit 332a (first-direction filtering means) for performing face extraction processing of the plane along the X direction by filtering the X direction (first direction) on the three-dimensional X-Y-Z orthogonal coordinates system for example, a Y-directional filtering unit 332b (second-direction filtering means) for performing face extraction processing of the plane following the Y direction by filtering the Y direction (second direction), a Z-directional filter processing unit 332c (third-direction filtering means) for performing face extraction processing of the plane along the Z direction by filtering the Z direction (third direction), and a calculating unit 333 (computing means) for calculating the sum of squares of the output from the filtering results of these directions each being processed, or calculating the square root of the sum of squares (or calculating vector length).

The X-directional filtering unit 332a is formed of a high-pass filter (HPF, or a low-cut filter), such as a Sobel filter or the like. The Y-directional filtering unit 332b and Z-directional filtering unit 332c are also formed of Sobel filters or the like, as with the X-directional filtering unit 332a.

Following converting the collected ultrasound sampling volumes into voxel volumes with the digital scan converter 29, face extraction filtering is performed with the face extraction filtering unit 33 having such a configuration,.

The face extraction filtering unit 33 is preferably configured of linear filters capable of disassembling voxel volumes with respect to each dimension, so that filtering is performed with regard to each direction, and following the filtering, the vector components are calculated based on the disassembled components.

The face components are the portions where the intensity value of the image suddenly changes, and of the echoes reflected from the region of an parenchymatous organ, the portions corresponding to the face components have high-frequency components, so that face components can be extracted by using a high-pass (enhancing) filter or a band-pass filter having noise reduction functions for composing the face extraction filtering unit 33, thereby creating an image with the face components enhanced. Note however, that various types of filters can be used for the filters.

Though the embodiments describe the usage of the filter, i.e., the way of face extraction and the way of its use, is used, as being extraction of face components by filtering a B/W volume which is three-dimensional volume data generated from the output of the echo processor 27, but the present invention is by no means restricted to this, and the following can be carried out with each embodiment, as well.

1) To perform filtering for face extraction on only one of B/W volume data (three-dimensional distribution information representing the tissue structure of the subject: three-dimensional volume data generated from the output of the echo processor 27) and color volume (three-dimensional distribution information representing the properties of moving objects in the subject: three-dimensional volume data generated from the output of the flow processor 28), and rendering the extracted face information (component) and the volume regarding which extraction was not performed, to generate image information for diagnosis.
2) To filter both the B/W volume data and color volume data to extract face information, and perform rendering to obtain three-dimensional image information.
3) An arrangement also may be made wherein a filter for extracting face information from the B/W volume data and a filter for extracting face information from color volumes are each weighted (or, filter coefficients may be adjusted) and means for adjusting the weighting, i.e., means for changing filtering conditions, are provided, and enabling the conditions of filtering to be changed by the means while actually viewing the image, thereby obtaining an even better image.

In the case of 3), the states of 1) and 2) above can be created by arranging for the weighting coefficients to be variable between 0 (no filter effects, i.e., through-pass) to 1 (state wherein filter is 100% effective). Performing filtering by such face extraction filtering allows, for example, the boundary between the full portions and cavities in parenchymatous organs to be displayed with enhancement, thereby visualizing cavities and tube structures more clearly. Examples of internal organs which would fall under this category include the liver (visualizing each of the hepatic veins, portal vein, and aorta), the gall bladder, and so forth.

Now, with a three-dimensional filter such as in the present embodiment, two-dimensional filtering is performed by dividing in each of the X, Y, and Z directions, such that filtering is performed by disassembling one-dimensionally in steps, i.e., first, the X-direction is subjected to filtering, then the Y-direction is subjected to filtering, and further the Z-direction is subjected to filtering. This allows three-dimensional filtering to be performed.

Regarding one direction, a Sobel filter has a 3 by 3 two-dimensional filter, for example, and with regard to the number of samples (taps), in the event of disassembling into each direction a high-pass filter of 3 by 3 = 9 taps for each direction is used to linearly filtering each of the three directions X, Y, and Z, thereby performing three-dimensional filtering.

The output of the Sobel filter reflects the magnitude of the face component in the processing direction, and the normal direction on the plane at the sample point of interest can be represented as a vector notation having as components thereof the output of the three directions X, Y, and Z.

That is to say, in the event of using the 3 by 3 Sobel filters 332a, 332b, 332c independently in the X, Y, and Z directions, the calculating unit 333 outputs the sum of squares of each output. Further, since the range of the output values is great if left in this way, the output of the calculating unit 333 may be the square root of the sum of squares, if necessary.

In this manner, image rendering by VR (volume rendering) can be performed on voxel format volumes which are the output of face extraction filtering processing, with the three-dimensional rendering engine 37.

The configuration of the face extraction filtering unit 33 is not restricted to the case described above, and may be configured of a three-dimensional filter capable of performing filtering on each of the three directions, front and behind the sample of interest, left and right thereof, and above and below. That is, only the front and behind, left and right, and above and below need to be viewed for detection of the presence of face components, in the simplest form, a configuration may be employed which uses the surrounding six samples. In addition to the above, a configuration may be employed which takes all 26 samples surrounding a particular sample of interest for computation, including the samples in all diagonal directions. Increasing the number of samples thus stabilizes the face extraction processing. Here, in the event that the face extraction filtering unit 33 is configured to disassemble voxel volumes with respect to each of the X, Y, and Z directions, two-dimensional filtering is used for each, however, in the event of performing three-dimensional computation with surrounding samples, a filter having a different configuration from that used for normal two-dimensional filter is used.

(Sobel filter)

The face extraction filtering unit 33 performs processing for independently applying 3 by 3 two-dimensional Sobel filters 332a, 332b, 332c to each of the X, Y, and Z directions, for example.

Now, assembling that $f(I, j, k)$ represents a pixel values (luminance or intensity) on $(I, j, k)$ coordinates in a digital image, for example, the Sobel filters have a 3 by 3 filter $g_{x3}(i, j, k)$ to be applied in the X direction, a 3 by 3 filter $g_{y3}(i, j, k)$ to be applied in the Y direction, and a 3 by 3 filter $g_{z3}(i, j, k)$ to be applied in the Z direction, each generating output defined by the following expressions.

$$g_{x3}(i, j, k) = f(i + 1, j + 1, k) + (+2) f(i + 1, j, k) + f(i + 1, j - 1, k) + (-1) f(i - 1, j + 1, k) + (-2)$$

$$f(i - 1, j, k) + f(i - 1, j - 1, k)$$

$$g_{y3}(i, j, k) = f(i + 1, j + 1, k) + (+2) f(i, j + 1, k) + f(i - 1, j + 1, k) + (-1) f(i + 1, j - 1, k) + (-2)$$

$$f(i, j - 1, k) + (-1) f(i - 1, j - 1, k)$$

$$g_{z3}(i, j, k) = f(i, j + 1, k + 1) + (+2) f(i, j, k + 1) + f(i, j - 1, k + 1) + (-1) f(i, j + 1, k - 1) + (-2)$$

$$f(i, j, k - 1) + (-1) f(i, j - 1, k - 1)$$

As the square root of the sum of squares of each output is calculated at the calculating unit 333, so the output F(i, j, k) thereof is

$$F(i, j, k) = (g_{x3}\ (i, j, k) \times g_{x3}\ (i, j, k) + g_{y3}\ (i, j, k) \times g_{y3}\ (i, j, k) + g_{z3}\ (i, j, k) \times g_{z3}\ (i, j, k))^{1/2}.$$

Where, f(i - 1, j - 1, k), f(i - 1, j, k), f(i - 1, j + 1, k), and so forth in the filter applied in the X-direction, are pixel values of the eight samples (voxels) near the sample of interest (i, j, k). FIG. 3A illustrates the array of the eight samples (voxels) in the image. The sample pixel value f(i, j, k) representing the voxel of the position (i, j, k) is generated from the adjacent voxel value positioned on the previous line {f(i - 1, j - 1, k), f(i - 1, j, k), f(i - 1, j + 1, k)} and the adjacent voxel value positioned on the same line {f(i, j - 1, k), f(i, j + 1, k)} and the adjacent voxel value positioned on the next line {f (i + 1, j - 1, k), f (i + 1, j, k), f (i + 1, j + 1, k)}, according to the aforementioned expressions.

The same computation as performed in the X direction using the nearby eight voxels is performed for the Y direction and the Z direction, as shown in FIGS. 3B and 3C. Note that filtering as referred to here means obtaining the sum of the product of multivalue image data values and filter values, and storing the absolute value thereof as a value obtained as the result of filtering.

Thus, values for the outline can be obtained from output having transmission in an arbitrary direction (horizontal, vertical, or diagonal direction)

(Smoothing filter)

The smoothing filter processing unit 31 is for performing smoothing at portions where steep face components appear in the original image, to prevent noise components contained in the input image from being recognized as face components, and comprises a median filter 331 which performs three-dimensionally-configured filtering for nearby six samples for example, in the X, Y, and Z directions, as shown in FIG. 2, for example.

The median filter 331 functions as a median filter for performing median extraction, which makes reference to the ultrasound image, compares nearby image data values for each sample position, and updates the value of the sample of interest so that the sample data of a middle value is set as the new value of a sample of interest, thereby removing speckle noise and the like contained in the ultrasound image.

Description of one example with the present embodiment will be made for a case of substituting the value at a sample of interest position with the median of the seven samples (seven taps) of the nearby six samples and itself.

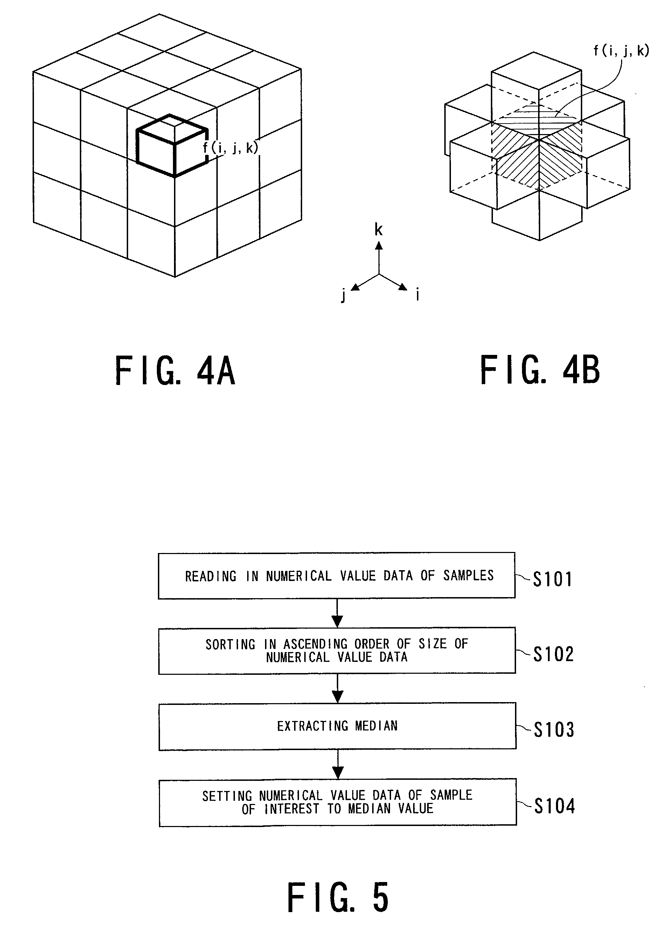

For example, FIG. 4A illustrates a sample of interest surrounded by total of 26 nearby samples, and as shown in FIG. 4B, with median filtering for the nearby six samples above and below (k direction) and left and right (i direction and j direction) of the sample of interest f(i, j, k), which makes for a total of seven samples (seven taps) including the pixel of interest itself, the following computation is performed for image data regarding which the median is extracted for seven numerical data sets.

For example, with the numerical data for the image that has been provided to the sample f(i, j, k) is 150, the numerical data provided to the sample f(i, j - 1, k) is 14, the numerical data provided to the sample f(i, j + 1, k) is 15, the numerical data provided to the sample f(i + 1, j, k) is 15, the numerical data provided to the sample f(i - 1, j, k) is 15, the numerical data provided to the sample f(i, j, k + 1) is 16, and the numerical data provided to the sample f(i, j, k - 1) is 16, almost all samples have numerical data between 14 and 16, but f(i, j, k) has numerical data of 150, which is not close to the values of the surrounding data, so it can be understood that this is noise.

In the event of correcting the value of f(i, j, k) using the median filter 331, the data of the sample f(i, j, k) and the surrounding nearby six samples, making a total of seven sets of data, are scrutinized. Arranging these in ascending order of size, the numerical values are 14, 15, 15, 15, 16, 16, and 150. Among them, the fourth value, i.e., the value positioned at the center of the data is called the median, and in this event is 15. Accordingly, this median 15 is used as the data for the sample f(i, j, k). Image processing carried out by applying the above operations to all samples is called median filtering in the present embodiment. Applying the median filter 331 to the image information removes the noise in this way.

In this manner, the median filter 331 performs the processing of reading in the sample of interest and the surrounding nearby six samples for a total of seven sets of numerical value data, which are arranged in ascending or descending order of size, and the median is extracted, thereby executing filtering from the first sample in the image data volume, and applying this to the entire image space, thus performing smoothing the image. In other words, as shown in FIG. 5, the numerical value data of the sample is read in (step S101), and sorted in ascending order of size of numerical value data (S102), from which the median is extracted (S103). The numerical value data of the sample of interest is set to the median value (S104).

Using the median filter allows excellent images to be obtained as compared with methods which average with the surrounding data for example, from the viewpoint of degree of noise removal and preservation of image outline and so forth, so that noise and isolated points can be removed without blurring the object.

As for the configuration of the median filter 331, a configuration may be used wherein the value is substituted with the median of the sample of interest itself and the nearby 26 samples making to a total of 27 samples. In this case as will, the above processing is performed for all sample positions within the volume. In the event that no nearby sample exists at the face of the volume, this is substituted with the value of the sample position of interest. Or, a configuration may be performed wherein the computation itself is not executed, and the sample value is used as the output value as it is.

In this manner, reduction of noise and the like can be effected by introducing the smoothing filtering unit 31 in addition to the face extraction filtering unit 33.

(Processing procedure)

The configuration of the ultrasound diagnosis apparatus 1 according to the present embodiment is as described above, and operates as described below.

Generally, the ultrasound probe 12 is operated manually or mechanically for scanning, to collect a three-dimensional volume.

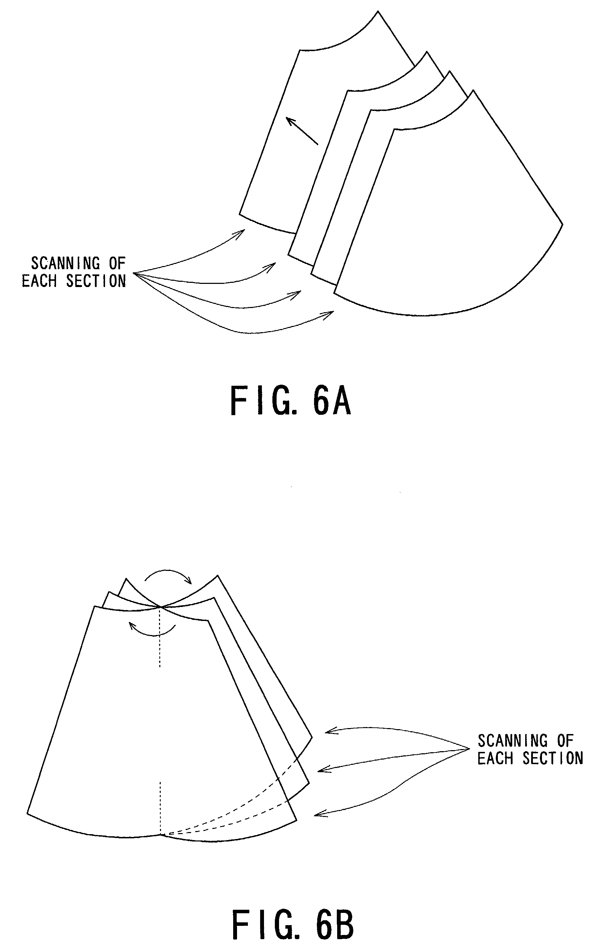

FIG. 6A explains a scan technique by which a section to be scanned is shifted along a perpendicular direction to the section during its scanning operation. Meanwhile, FIG. 6B explains another scan technique used in such a manner that a section to be scanned is shifted to rotate about its central axis during its scanning operation.

The host CPU 17 determines the ultrasound scanning mode and the display mode in compliance with input from the operating unit 18, and sets parameters necessary for the units such as the real-time controller (RTC) 16 before scanning. Upon finishing setting of the necessary parameters, a scan start command is issued to the real-time controller (RTC) 16.

The real-time controller (RTC) 16 transmits high-voltage pulse generation timing signals and delay control data, necessary for irradiation from the ultrasound probe 12, to the transmission unit 14. Based on the signal and control data, the transmission unit 14 applies high-voltage pulse signals to the ultrasound probe 12, so that ultrasound signals are irradiated into the body. The reflected waves from the organs within the body are subjected to noise removal and amplitude amplification at the reception unit 22, converted into digital data at unshown A/D converter, and subjected to phasing addition processing at the phasing adder 24, thereby generating ultrasound beam data. The detection circuit 26 performs quadrature phase detection processing as to the ultrasound beam data, so as to convert then into a complex format sample having phase information.

The output from the detection circuit 26 is shunted to either the echo processor (EP) 27 or the flow processor (FP) 28, depending on the image display mode. The echo processor (EP) 27 performs envelope detection and performs processing for forming pictures of reflection wave intensities from the tissue. On the other hand, the flow processor (FP) 28 extracts Doppler signals using autocorrelation functions, and computes the velocity of the blood flow and the like, and the dispersion, the power, and so forth, thereof. Note that these ultrasound samples may be referred to as "ultrasound vector data" to facilitate description.

The ultrasound vector data is then converted into voxel-format volume data in the orthogonal X-Y-Z axes at the digital scan converter (DSC) 29 and the volume generator 30.

The smoothing filtering unit 31 performs smoothing on the voxel-format volume data, using various types of filters such as a median filter using nearby six samples or a median filter using nearby 26 samples or the like.

Subsequently, the face extraction filtering unit 33 performs two-dimensional filtering on the voxel volume data formed of voxels (samples) with a Sobel filter or the like in the X direction, two-dimensional filtering with a Sobel filter or the like in the Y direction, and two-dimensional filtering with a Sobel filter or the like in the Z direction, and calculates the square root of the sum of squares of each of the output results, thereby performing filtering of the sample of the region of interest.

Then, at the three-dimensional rendering engine 37, the voxel volume is subjected to volume rendering, and a three-dimensional rendering image which has been smoothed and rid of speckle noise, wherein the internal structures can be seen by face extraction, is displayed on the display unit 38 such as a CRT or the like.

Thus, with the present embodiment, for example, the liver U1 may be displayed on the display unit according to a normal mode as shown in FIG. 7A, the internal structure U2 of the liver U1 can be clearly displayed as shown in FIG. 7B by changing a mode to an internal structure observing mode.

With regard to the display format of the three-dimensional image displayed on the display unit 38, in addition to the first three-dimensional image displaying the internal structures of parenchymatous organs, such as the cavital structures within the liver for example, as described above, face enhancement filtering may be applied to the image obtained by the color Doppler method.

That is, displaying an image wherein a face enhancement filter has been applied to a three-dimensional blood vessel image which is made possible to be displayed, with the flow processor 28, enables a display to be made wherein the organ can be seen through, and a blood vessel image can be viewed therein.

Similarly, as for locations where there is no blood flow such as with the liver, the gall bladder or the like, using the color Doppler method does not bring out a blood vessel image, however, a blood vessel image can be displayed even for places with no blood flow, by performing face enhancing (face component extraction) filtering processing as with the present embodiment. Also, data corresponding to blood vessels may be displayed in a superimposed manner.

According to the present invention as described above, the blood vessels and cavitary structures within an parenchymatous organ can be comprehended in a more three-dimensional manner, without performing volume operations such as clipping, with a face extraction filter. Further, removal of speckle noise and the like can be performed by a smoothing filter.

Second Embodiment

Next, a second embodiment according to the present invention will be described with reference to FIG. 8. In the following, the configurations which are essentially the same as those in the first embodiment will be omitted from the description. The components which have generally the same functions and configurations will be denoted with the same reference numerals as in the first embodiment, and redundant description thereof will be omitted unless necessary, so basically only the differing parts will be described. FIG. 8 is a functional block diagram illustrating an example of a configuration of the ultrasound diagnosis apparatus according to the present embodiment.

With the first embodiment, the smoothing filter was configured as a three-dimensional filter using a predetermined number of surrounding samples, in the meantime, with the present embodiment, the smoothing filter is disassembled into the X, Y, and Z directions, respectively, and processing is carried out by two-dimensional filters.

Specifically, the smoothing filtering unit 31A according to the present embodiment comprises a median filter 334a which performs filtering on an (x, y) plane, a median filter 334b which performs filtering on a (y, z) plane, and a median filter 334c which performs filtering on a (z, x) plane, as shown in FIG. 8.

On the other hand, the smoothing filtering unit 33A has Sobel filters 335a, 335b, 335c, and a vector length calculating unit 336, the same arrangement as in the first embodiment.

In this case, the processing is divided and performed two-dimensionally, with the median of 3 by 3 samples on the x-y plane including the sample of interest being calculated by the median filter 334a, the median of 3 by 3 samples on the y-z plane calculated by the median filter 334b, and the median of 3 by 3 samples on the z-x plane calculated by the median filter 334c.

Subsequently, the output of each median filter 334a, 334b, and 334c, is subjected to processing in mutually independent directions by the Sobel filters 335a, 335b, and 335c, which process the same planes respectively, thereby extracting face components. Calculation of the vector length at the calculating unit 336 is the same as with the above-described processing.

According to the present embodiment thus described, processing by smoothing filters is performed for each direction, so in the event that a two-dimensional array probe is used, noise removal capabilities are improved by performing three-dimensional filtering in the X, Y, and Z directions since speckle noise and the like occurs differently according to the direction, thereby improving image quality.

Besides, at the time of performing processing using Sobel filters, 3 by 3 samples are loaded to the computing device, so that the processing can be simplified by parallel processing by median filters.

(Modification of face extraction filtering processing unit)

While arrangements have been described in the first and second embodiments regarding an example of the face extraction filtering unit 33, 33A wherein Sobel filters are used for the direction X, Y, and Z, an arrangement maybe made wherein the sum of absolute differences with the surrounding six samples of the sample (voxel) of interest is taken. Further, the weighted average using the distance from the sample (voxel) of interest may be taken. Specific examples are as follow.

For example, detection of the portion where the intensity value of the image suddenly changes may be performed with primary or secondary differential Laplacian filters, spatial derivative filters, Volsen filter, Robert filter, Range filter, or the like. At this time, whether to disassemble in each direction and use as a combination, or whether to not disassemble in each direction and use a three-dimensional configuration, is optional. Also, in the event that disassembling in each direction, different types of filters may be used in each direction. Further, the configuration may involve filters is a particular disassembled direction being applied multiple times.

(Modification of smoothing filtering unit)

Note that the three-dimensional processing of the smoothing filter may be such that is only in one direction.

Processing techniques by the smoothing filtering unit include a simple average processing method wherein the average of values of samples within a predetermined region around the sample is obtained, and this average value is set to the value of the center sample, a method using median filter wherein the median of the values of the predetermined region is set to the center pixel value, a method using a face-saving filter (V filter) wherein the above predetermined region is divided into further smaller regions and the dispersion per small region is obtained so as to set the average value of the small region of the smallest dispersion to the center pixel value, and a method wherein image signals are subjected to Fourier transform, and following removal of high spatial frequency components corresponding to the noise components, inverse Fourier transform is performed, and so forth can be employed.

In addition, a moving average filter taking the average intensity of values of the near samples may be used. Further, a filter having the nature of a high-cut filter (a low-pass filter) is sufficient for smoothing, so depending on properties, a Butterworth filter, Chebyshev or elliptic filter, or a Gaussian filter may be used.

Third Embodiment

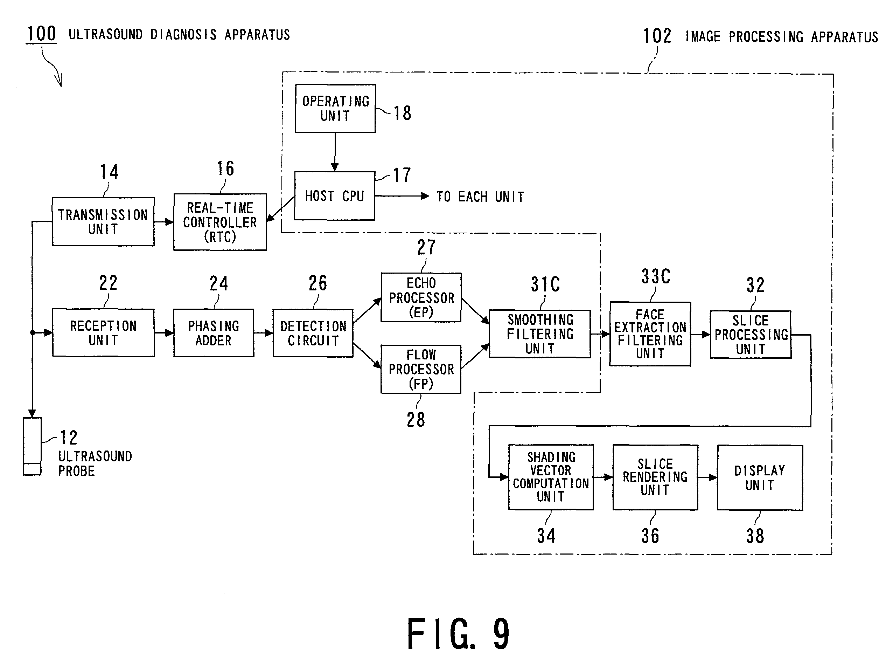

Next, a third embodiment according to the present invention will be described with reference to FIG. 9. In the following, the configurations which are essentially the same as those in the previous embodiments will be omitted from the description. The components which have generally the same functions and configurations will be denoted with the same reference numerals as in the previous embodiments, and redundant description thereof will be omitted unless necessary, so basically only the differing parts will be described. FIG. 9 is a functional block diagram illustrating an example of a configuration of the ultrasound diagnosis apparatus according to the present embodiment.

While the previous embodiments disclosed a configuration wherein face extraction filtering is performed on voxel volumes, the present embodiment discloses a configuration for performing face extraction filtering on radially-extending volume data.

(Configuration of ultrasound diagnosis apparatus)

FIG. 9 illustrates a block diagram of the configuration of the ultrasound diagnosis apparatus according to the present embodiment. As shown in FIG. 9, the ultrasound diagnosis apparatus 100 according to the present embodiment comprises a ultrasound probe 12, a transmission unit 14, a real-time controller (RTC) 16, a host CPU 17, a operating unit 18 which makes up a user interface, a reception unit 22, a phasing adder 24, a detection circuit 26 which is a detection unit, an echo processor (EP) 27, a flow processor (FP) 28, a smoothing filtering unit 31, a face extraction filtering unit 33, a slice processing unit 32, a shading vector computation unit 34, a slice rendering unit 36, and a display unit 38 such as a CRT or the like. Note that reference numeral 102 denotes the configuration of the image processing apparatus.

The ultrasound probe 12 is a two-dimensional ultrasound array probe wherein piezoelectric transducers are disposed in a matrix shape, so as to collect volume data in a radially-expanding shape from the surface of the probe, by ultrasound scanning. Volume data in a similar shape may obtained by swinging a sector probe. The spatial position of the collected ultrasound samples are represented using collection coordinates corresponding to the scan shape of the ultrasound scan. Since a method using polar coordinate having three parameters of R, $\theta$, and $\psi$ as collection coordinates is most suitable with the embodiment,, the following description will be made with regard to using polar coordinates.

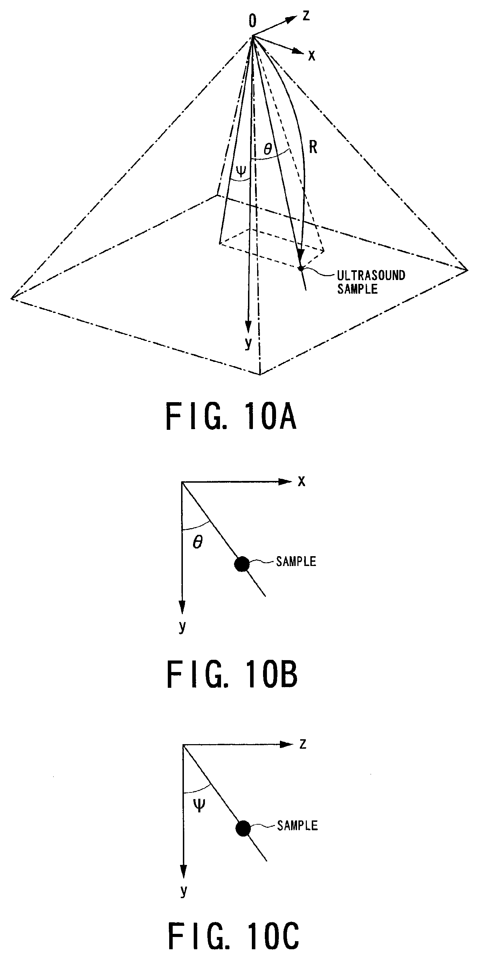

FIG. 10A illustrates the geometric shape of a volume collected using the ultrasound probe 12. Point O is the center of the surface of the ultrasound probe 12, and a line perpendicular to the probe surface at point O is defined as the Y axis. Also, the X axis and Z axis mutually perpendicular and perpendicular to the Y axis are set as shown in FIG. 10A. Since the entire ultrasound beam is formed radially from the point O, so the ultrasound sample data making up the ultrasound beam is most suitably represented by polar coordinates. Accordingly, the distance from the point O to an ultrasound sample is defined as R, and as shown in FIGS. 10B and 10C, the angle between the projected ultrasound beam obtained by projecting the ultrasound beam on the X-Y plane and the Y axis is defined as $\theta$, and similarly the angle between the projected ultrasound beam obtained by projecting the ultrasound beam on the Z-Y plane and the Z axis is defined as $\psi$. Consequently, the relation between the polar coordinates and the orthogonal coordinates in this case is as follows.

Conversion from orthogonal coordinates system to polar coordinates system:

$$R = (x^2 + y^2 + Z^2)^{\frac{1}{2}}$$

$$\theta = \tan^{-1}(x/y)$$

$$\psi = \tan^{-1} (z/y)$$

Conversion from polar coordinates system to orthogonal coordinates system:

$$x = R \times \tan\theta \times \{1/ (1 + \tan^2\theta + \tan^2\psi) \}^{1/2}$$

$$y = R/(1 + \tan^2\theta + \tan^2\psi) \}^{1/2}$$

$$z = R \times \tan\psi \times \{1/(1 + \tan^2\theta + \tan^2\psi) \}^{1/2}$$

where, $\times$ indicates multiplication.

In FIG. 9, the real-time controller (RTC) 16 performs timing control for transmission and reception of ultrasound signals, based on the scan control parameters. The scan control parameters used there are those which the host CPU 17 has obtained based on input by the user via the operating unit 18. Though not shown in the drawings, the real-time controller 16 internally has a timer and sequence circuit or program therein, in compliance with the scan control parameters set by the host CPU 17, to operate the timer based on information such as a ultrasound collection modes such as B/W or color Doppler scanning, and an ultrasound data collection repetition cycle, thereby generating ultrasound transmission reference timing signals cyclically generated based on the output of the timer.

The beam address indicating the position within the volume of the ultrasound data collected is determined by the angles θ (row) and ψ (column) to a direction perpendicular to the probe surface of the ultrasound probe 12 and in mutually orthogonal directions. In other words, the ultrasound beam can be represented as [row beam address, column beam address] in the two-dimensional disposition format.

The real-time controller (RTC) 16 generates, in addition to the beam address, information necessary for processing, such as beam type for identifying whether the ultrasound beam is B/W data or color Doppler data, data collection distance, as header information. The generated header information is added to the data at the later-described reception unit 22, and is transmitted to the units for performing the subsequent processing along with the data.

The smoothing filtering unit 31C then performs smoothing on the ultrasound volume data from the flow processor (FP) 28 or the echo processor (EP) 27, and further, the data subjected to smoothing by the smoothing filtering unit 31C is subjected to face extraction (face component enhancing) processing. Thus, following subjecting the ultrasound volume data to smoothing and face extraction processing, a three-dimensional image is generated at the slice processing unit 32, shading vector computation unit 34, slice rendering unit 36, and so forth.

The host CPU 17 successively judges the operation inputs successively made by the user via the operating unit 18 as to the three-dimensional images, such as rotation operations for the volume, and performs controls regarding display of the three-dimensional image by setting necessary parameters to the later-described slice processing unit 32, shading vector computation unit 34, and slice rendering unit 36.

(Slice processing unit)

Though not shown in FIG. 9, the slice processing unit 32 has memories and a control circuit for rearranging the ultrasound sample data input from the echo processor (EP) 27 or the flow processor (FP) 28, and performs rearranging processing of the ultrasound sample data based on the slice configuration information set by the host CPU 17, thereby outputting a data group configured of all ultrasound sample data on a slice face (hereafter referred to as "ultrasound slice data").

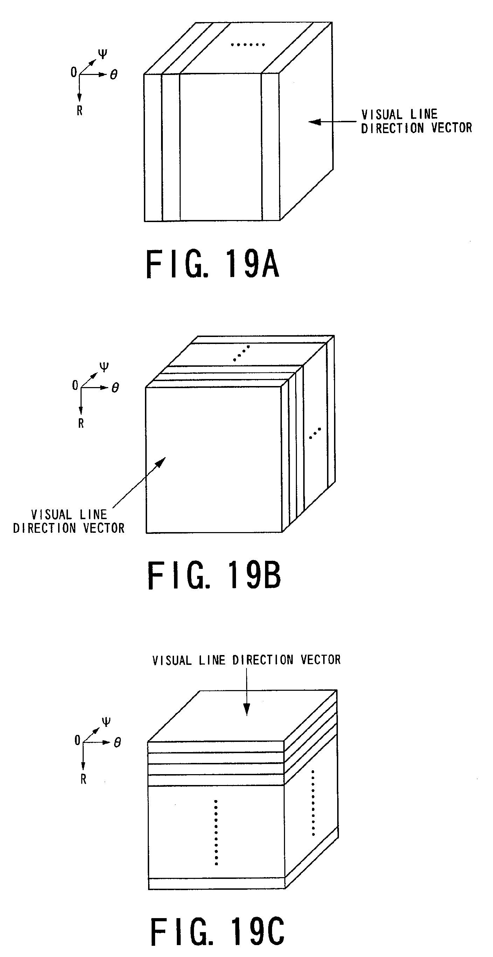

Note that as shown in FIG. 19, a slice face is restricted to one of the following: with the same distance R from the point O, with the same deviation angle θ, or with the same deviation angle ψ; and forms a plane or a spherical surface.

FIG. 19A illustrates the R-ψ slice face with the same θ, FIG. 19B illustrates the R-θ slice face with the same ψ, and FIG. 19C illustrates the θ-ψ slice face with the same R. The axis among the X axis, Y axis, and Z axis which is the closest to parallel with the visual line direction vector is obtained, and in the event that the X axis is the closest to parallel, the R-θ slice face is taken, in the event that the Y axis is the closest to parallel, the ψ-θ slice face is taken, and in the event that the Z axis is the closest to parallel, the R-θ slice face is taken.

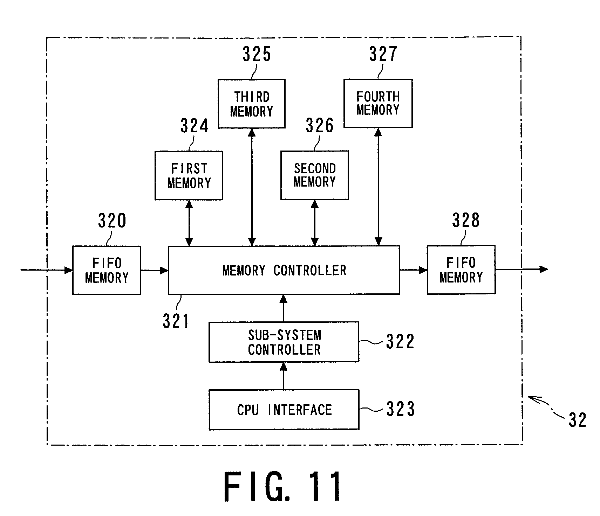

As shown in FIG. 11, the specific configuration of the slice processing unit 32 comprises FIFO (First-in First-out) memory 320 and 328, a memory controller 321, a sub-system controller 322, a CPU interface 323, a first memory 324, a second memory 325, a third memory 326, and a fourth memory 327.

The memory controller 321 performs control so as to divide the memory cycle into the two cycles of reading and writing which are executed alternately, in order to simultaneously perform writing and reading of data to and from the first memory 324 through the fourth memory 327.

The ultrasound sample data input from the echo processor (EP) 27 or the flow processor (FP) 28 is temporarily stored in the FIFO memory 320. The memory controller 321 deciphers the beam position information within the header information attached to the ultrasound sample data, and writes data corresponding to the row/column beam address to the first memory 324 through the fourth memory 327. The first memory 324 through the fourth memory 327 form a grid within a logical three-dimensional memory space, and are configured so as to store two sets of ultrasound volume data corresponding to (R, θ, ψ) in order to raise the speed of processing by simultaneously writing and reading.

Note that the first memory 324 and the second memory 325 store data corresponding to even beam addresses and data corresponding to odd beam addresses of first volume data respectively, and the third memory 326 and the fourth memory 327 store ultrasound sample data corresponding to even beam addresses and ultrasound sample data corresponding to odd beam addresses of second volume data respectively.

The sub-system controller 322 reads out the data from the first memory 324 through the fourth memory 327 based on the read control parameters set by the host CPU 17 via the CPU interface 323.

Data reading is performed so as to configure ultrasound slice data of a slice face parallel to one of the R-θ slice face (the face parallel to the R axis and the θ axis), the θ-ψ slice face (the face parallel to the θ axis and the ψ axis), and the ψ-R slice face (the face parallel to the ψ axis and the R axis). In the event of configuring the R-θ slice face, first, data is read out from the face portion of the ultrasound volume data in the R direction.

After reading out one beam worth of data, the row addresses are read out with priority, and the column address is changed at the point that the row address reaches the face portion of the ultrasound volume data. In the event of configuring the R-ψ slice face, the column addresses are read out with priority instead, and the row address is changed at the point that the column address reaches the face portion of the ultrasound volume data. In the event of configuring the θ-ψ slice face, R has the lowest priority for reading, so the row/column addresses are sequentially changed, and the R-direction address is changed at the point that one slice worth of data has been read out.

The data read out according to the above method comprises a slice face according to one of R-θ, θ-ψ, ψ-θ, and is sequentially transmitted to the subsequent unit with the timing being adjusted at the FIFO memory 328.

(Shading vector computation unit)

The shading vector computation unit 34 obtains three-dimensional normal vectors necessary for shading, by computing the gradient of intensity values which each ultrasound sample data has, based on the ultrasound slice data output by the slice processing unit 32.

FIGS. 12A through 12C are conceptual diagrams for describing the conversion processing by the shading vector computation unit 34 for converting normal vectors on a polar coordinates system into those on an orthogonal coordinates system. FIG. 12A illustrates ultrasound slice data on polar coordinates that are input to the shading vector computation unit 34, with a blood vessel running linearly on the R-θ slice face, and with an intensity gradient as to the adjacent tissue (the arrows in the drawing) present. FIG. 12B illustrates the ultrasound slice data on an orthogonal coordinates system that has been represented on the polar coordinates system shown in FIG. 12A, with a blood vessel running concentrically at a equal distance from the start point of the ultrasound beam, and with an intensity gradient as to the adjacent tissue present. FIG. 12C is a conceptual diagram of the output data of the shading vector computation unit 34, with the shading vector computation unit 34 outputting normal vectors on the orthogonal coordinates corresponding to each point on the slice face represented on the polar coordinates system of R, θ, and ψ (hereafter referred to as normal vector slice data).

Since the ultrasound sample data input to the shading vector computation unit 34 is positioned on the polar coordinates (R, θ, ψ), the concentric blood vessel is represented as a straight line on the polar coordinates system as shown in FIG. 12A. Consequently, the intensity gradients on the polar coordinates system all face the same R direction, and are represented as mutually parallel vectors. That is, the obtained normal vectors are all in the same direction on the polar coordinates system. On the other hand, the logical image generation space where three-dimensional images are generated is an orthogonal coordinates system (X, Y, Z), so the blood vessel should be displayed as a curve having a certain curvature, with the intensity gradient oriented toward the start point of the ultrasound beam, as shown in FIG 12B.

Accordingly, the shading vector computation unit 34 computes the normal vectors according to expressed by orthogonal coordinates as follows. First, the necessary ultrasound sample data is stored in the memory. Next, the necessary ultrasound sample data is read out from the memory, thereby yielding the gradient of intensity values by difference. Finally, the normal vectors at the points where the gradient has been calculated, expressed by polar coordinates system, are converted into normal vectors expressed by orthogonal coordinates system. For the calculation of the reflected light ray amount toward the visual line direction in the three-dimensional rendering image generation, normalization processing is performed wherein the length of the normal vector is set to 1 after coordinates conversion, since computation is facilitate by having the normal vectors normalized.

Further, weighted addition processing with nearby normal vectors may be performed in order to make the normal vectors less susceptible to noise called speckles, commonly known in image forming techniques using ultrasound.

The orthogonal-coordinates normal vectors are computed from the ultrasound sample data making up the slices sequentially input from the slice processing unit 32, and accordingly make up normal vector slice data making up the same slices as the input. Also, the normal vector slice data is displaced in the three-dimensional space, and a set of the normal vectors corresponding to one volume is referred to as a normal vector volume.

The following is the detailed configuration of the shading vector computation unit 34.

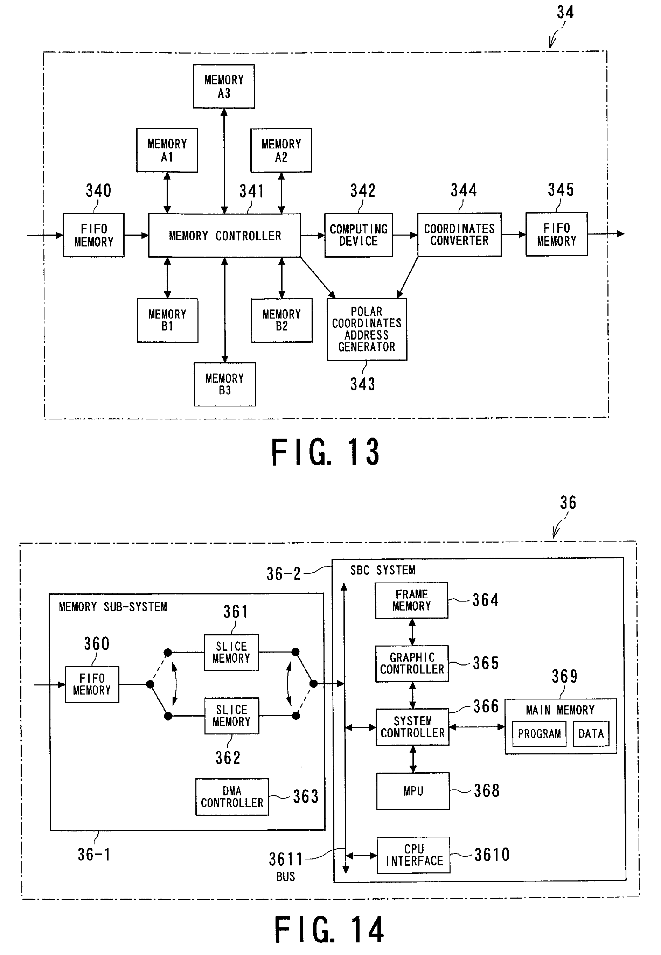

As shown in FIG. 13, the shading vector computation unit 34 comprises FIFO memory 340 and 345 functioning to buffer data exchange at the time of writing and reading data, memory A1, A2, A3, B1, B2, and B3 for holding samples nearby a sample of interest, a memory controller 341 for controlling each of the memory, a computing device 342 for calculating the normal vectors of the face detected by the intensity gradient, a polar coordinates address generator 343 for calculating the polar coordinates position of the ultrasound sample data of interest corresponding to the address, and a coordinates converter 344 for performing conversion of the normal vectors represented by polar coordinates into normal vectors represented by orthogonal coordinates, as well as performing normalization of the normal vectors.

The shading vector computation unit 34 performs normal vector computation processing necessary for shading, based on the ultrasound sample data input from the echo processor (EP) 27 or the flow processor (FP) 28.

(Input of ultrasound beam data)

First, the input ultrasound beam data is temporarily stored in the FIFO memory 340, and is written to one of the memory A1, A2, A3, B1, B2, and B3 under the predetermined control of the memory controller 341. The memory A1, A2, and A3 (memory A group) and B1, B2, and B3 (memory B group) are configured such that while one is performing writing processing, the other is performing reading processing, and the memory controller 341 controls such that the reading and writing switch each time collecting of a volume is completed.

Now, it is assumed that the memory A group is set to the write side. AT this time, the memory controller 341 obtains beam position information for determining the ultrasound beam position contained in the header information attached to the sample data, and outputs the write address and write control signals according to the beam number to one of the memory A1, A2, and A3. Which of the memory A1, A2, or A3 to write to is determined using the row beam address of the beam addresses.

As described above, the input ultrasound sample data is distinguished by the beam number represented by the column and row corresponding to the position in the three-dimensional volume. The memory to which writing is performed is sequentially switched, using the values of the row and column addresses which the input ultrasound sample data has.

Now, it is assumed that the ultrasound sample data for one ultrasound beam is configured of 1024 samples. In this case, the memory is selected according to the row address, and the offset within the selected memory is determined according to the column address. Adding the number of ultrasound sample data that have been written to the offset sequentially determines the final memory placement position for the sample. Thus, the input ultrasound sample data is placed in dispersed memory.

Thus, at the point that all of the ultrasound volume data has been collected and writing of the ultrasound vector data set to the memory A group has been completed, the reading/writing settings of the memory is switched by the memory controller, so that the memory B group is set to writing, and the memory A group to reading. For the subsequently-collected ultrasound volume data, the same processing is performed except that memory B1 is used instead of memory A1, memory B2 instead of memory A2, and memory B3 instead of memory A3.

(Read control of the memory controller)

Shading consists of taking a boundary face which a intensity gradient creates between the ultrasound sample data of interest and nearby ultrasound sample data as a face having an object of display, and calculating the reflected components of reflected light from the light source, thereby adding shading to the three-dimensional image. In order to obtain the intensity gradient, the ultrasound sample data nearby the ultrasound sample data of interest is necessary. Here, a method for obtaining the intensity gradient using $3 \times 3 \times 3 = 27$ samples including the ultrasound sample data of interest itself is used. With the method for reading out 27 samples per ultrasound sample data of interest, 27 times the amount of data reading as compared to data writing is necessary, so sequentially processing the nearby ultrasound sample data allows the ultrasound sample data that has been read out to be reused, thereby enabling the amount of memory reading to be reduced.

The memory controller 341 is arranged so as to be capable of controlling each memory at the same time, so that the nearby ultrasound sample data can be simultaneously read out from the memory A1, A2, and A3. For example, in the event of processing the ultrasound sample data with a row beam address of 10, the ultrasound sample data with row beam addresses of 9, 10, and 11 are simultaneously read out from the memory A1, A2, and A3.

The column address increased in increments of one at a time, so as to read out the data for the column beam address of interest and the one slice of data before and after. The necessary ultrasound sample data is sequentially read out in this manner, thereby obtaining the ultrasound sample data of interest and the nearby ultrasound sample data. The ultrasound sample data that has been read out is subjected to obtaining of difference of gradient of the intensity values of the ultrasound sample data at the computing device 342, thereby yielding normal vectors.

The coordinates converter 344 performs conversion of the normal vectors represented by polar coordinates output from the computing device 342 into normal vectors represented by orthogonal coordinates, as well as performing normalization of the normal vectors, which are output through the FIFO memory 345.

Thus, the difference between the intensity of the sample of interest at the center and the intensity of the samples surrounding the sample of interest is obtained, and in the event that the difference in intensity is great, a plane is viewed as existing at the center, and the direction which the plane is facing is represented by normal vectors. In the event that the intensity difference is great, normal vectors with large values are created, and in the event that the difference in the intensity is small, normal vectors with small values are created.

In order to see the angle as to the light source, the normal vectors are normalized to a normal vector length of 1, and shading processing corresponding to the direction of light is performed based on the angle between the normalized normal vectors and the light source vector from the light source.

Since the normal vectors before shading (normalization) change in size according to difference of the intensity, in the event that the difference in the intensity is great, normal vectors with large values are formed, and in the event that the difference in the intensity is small, normal vectors with small values are formed.

(Slice rendering unit)

To the slice rendering unit 36, ultrasound slice data is input from the slice processing unit 32, and normal vector slice data is input from the shading vector computation unit 34, and both are used to generate a three-dimensional volume rendering image.

As shown in FIG. 14, the slice rendering unit 36 is made up of a memory sub-system 36-1 and an SBC (single board computer) system 36-2, with both connected via a bus 3611 attached to the SBC system.

The memory sub-system 36-1 is configured of FIFO memory 360, slice memory 361 and 362, and a DMA (direct memory access) controller 363. The DMA controller 363 performs data transmission control within the memory sub-system 36-1. first, the DAM controller 363 performs temporary recording of the ultrasound slice data and the normal vector slice data input from the slice processing unit 32 or the shading vector computation unit 34, using the FIFO memory 360.

Next, the data recorded in the FIFO memory 360 is read out from the FIFO memory 360, and is recorded in the slice memory 361 which is made up of DRAM capable of recording a plurality of sets of slice memory. Upon recording data for the necessary slices, the data is read out from the slice memory 361, and is sent to the SBC system 36-2. The slice memory 361 and 362 assume a so-called double-buffer configuration, and while the slice memory 361 is transmitting the data to the main memory 369, and slice memory 362 records new data from the slice processing unit 32 and the shading vector computation unit 34.

The SBC system 36-2 comprises an MPU 368, system controller 366, main memory 369, a graphic controller 365, frame memory 364, a CPU interface 3610, and a bus 3611. The data sent from the memory sub-system 36-1 is sent to the data region of the main memory 369 via the bus 3611 and the system controller 366. The MPU 368 performs processing following the program stored in the program region separately provided within the main memory 369. The MPU 368 generates a three-dimensional image by cooperative action with the graphic controller 365 and temporarily stores the image into the frame memory 364. The graphic controller 365 reads out the three-dimensional image data based on the stipulated display timing signals, and transmits the data to the display unit 38.

The display unit 38 is configured of a CRT or LCD, and displays the three-dimensional image data generated at the slice rendering unit 36.

(Face extraction processing with present embodiment)

With normal image processing, the volume data is in the form of voxels, i.e., X-Y-Z orthogonal coordinates system data, while with ultrasound diagnosis devices, particularly with image processing using two-dimensional array probes, the volume data is in the form of a conical beam expanding in a radial fashion from a certain point, so data enters radially from the certain point. At this time, temporarily converting into voxels requires a time delay until displaying, so a technique wherein rendering is performed directly, is preferable. Accordingly, in such a case, the data is not temporality converted into orthogonal coordinates system data, rather, face extraction processing is performed in the R, θ, and ψ polar coordinates system.

Specifically, first filtering processing is performed with regard to the input data on the R, θ, and ψ polar coordinates system, using a smoothing filter. Next, second filtering processing is performed with a face extracting filter, with the image data that has been processed being overlaid one at a time using slices, and used in a combined manner.

At the face extraction filtering unit 33C at this time, filtering is performed by disassembling in each of the R, θ, and ψ directions, such that filtering is performed one-dimensionally in steps, i.e., for example, the R-direction is subjected to filtering, then the θ-direction is subjected to filtering, and further the ψ-direction is subjected to filtering. This allows three-dimensional filtering to be performed.

(Flow of collection of ultrasound volume data and image generating processing)

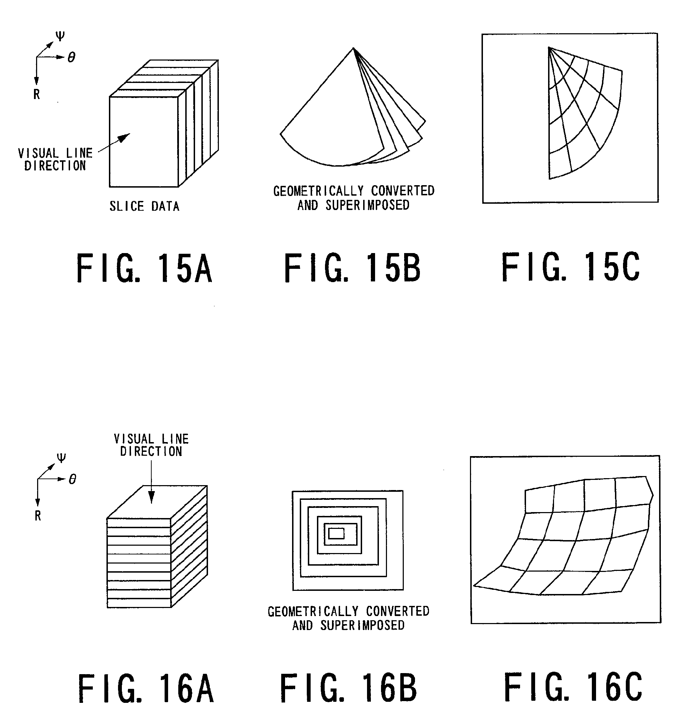

FIGS. 15A through 15C represent the concepts of the ultrasound volume data and the image generating processing of the ultrasound diagnosis apparatus 100 according to this embodiment.

FIGS. 15A through 15C describe a case wherein the visual line direction is the ψ-axial direction, with an ultrasound slice data group being generated from the obtained ultrasound volume data, and the ultrasound slice data being geometrically converted and superimposed by rendering processing, so as to generate a display image. FIGS. 16A through 16C describe a case wherein the visual line direction is the R-axial direction, with an ultrasound slice data group being generated from above the ultrasound volume data, and the ultrasound slice data being geometrically converted and superimposed by rendering processing, so as to generate a display image.

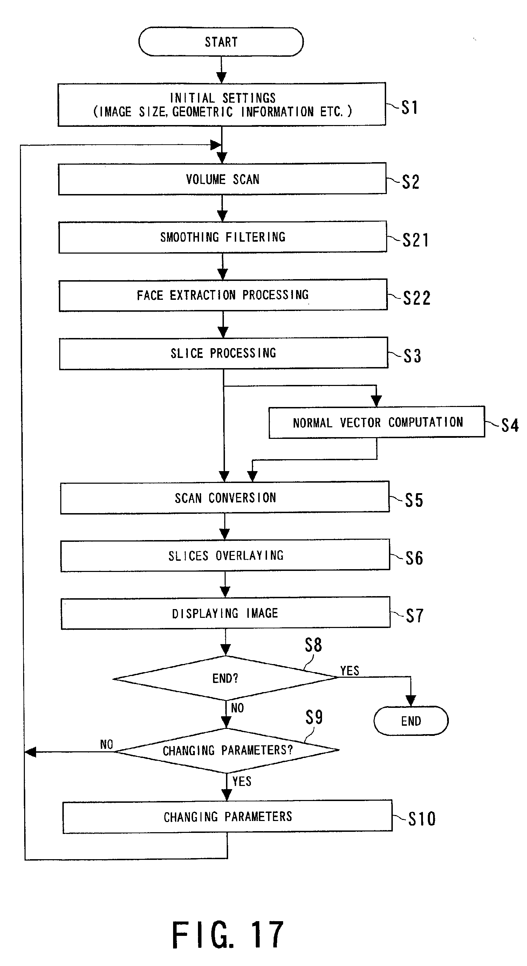

FIG. 17 is a flowchart conceptually illustrating the procedures for ultrasound volume collection and image generation with the ultrasound diagnosis apparatus 10 according to this embodiment.

First, as shown in FIG. 17, initial settings are made of each corresponding unit by control information set by the host CPU 17 beforehand, such as ultrasound volume collection conditions, display image size, visual line direction, geometric information, and so forth (step S1).

The initial settings may be made by a configuration wherein the setting are made automatically following turning on the electric power source, or wherein the user manually makes the settings via the operating unit 18.

Next, under the control of the real-time controller (RTC) 16, scanning of the ultrasound volume radially expanding from the surface of the ultrasound probe 12 is executed, and the volume data collected by the scan is subjected to the above-described processing at the reception unit 22, the phasing adder 24, the detection circuit 26, the echo processor (EP) 27, and the flow processor (FP) 28 (step S2).

Next, the smoothing filtering unit 31C performs smoothing processing using median filters or the like with regard to the ultrasound volume data output from the echo processor (EP) 27 and the flow processor (FP) (step S21).

Further, the face extraction filtering unit 33C performs face extraction processing with regard to the ultrasound volume data (step S22). At this time, the face extraction filtering unit 33C performs filtering one-dimensionally in steps upon disassembling, i.e., for example, the R-direction is subjected to filtering, then the θ-direction is subjected to filtering, and further the ψ-direction is subjected to filtering. This allows three-dimensional filtering processing to be performed.

The slice processing unit 32 takes the ultrasound volume data output from the echo processor (EP) 27 and the flow processor (FP) 28 and subjected to filtering such as smoothing and face extraction, and divides the ultrasound volume data into a plurality of ultrasound slice data groups parallel to one of the R-ψ slice face, the R-θ slice face, or the θ-ψ slice face, then outputs (step S3). The details of step S3 will be described later.

Next, the shading vector computation unit 34 computes the gradient of intensity values which each ultrasound sample data set has based on the ultrasound slice data group output from the slice processing unit 32, and obtains three-dimensional normal vectors necessary for shading, which are output as normal vector slice data (step S4).

The slice rendering unit 36 performs polygon processing using texture mapping to generate a three-dimensional image, based on the ultrasound slice data output by the slice processing unit 32 and the normal vector slice data output by the shading vector computation unit 34 (steps S5 and S6). In step S5, geometric processing including angle correction and enlargement/reduction for the final display is performed on the slice data group generated in step S4, and in step S6 opacity or color correction necessary for generating a three-dimensional image, and shading processing if necessary, is performed so as to generate an intermediate image, and the intermediate images are cumulatively added to generate an cumulative added image. This cumulative added image is the image wherein the ultrasound volume data is three-dimensionally projected. The display unit 38 displays the cumulative added image generated at the slice rendering unit 36 (step S7).

Following display, judgment is made regarding whether or not to end the processing (step S8). In the event of continuing the processing, judgment is made regarding whether or not there have been changes to display parameters including the visual line direction and so forth (step S9). In the event that there has been no change to the parameters, the flow returns to step S2 and the above-described series of processing is repeated. In the event that there have been changes made to the parameters, the necessary parameters are set to the respective units, and the flow returns to step S2.

Successively applying the processing to a plurality of volumes yields three-dimensional images in time-sequence, so that the moving state of organs, such as the walls and valves of the heart, or the moving state of the blood flow from a contrast agent or from color Doppler data, can be observed.

(Ultrasound slice data generation processing)

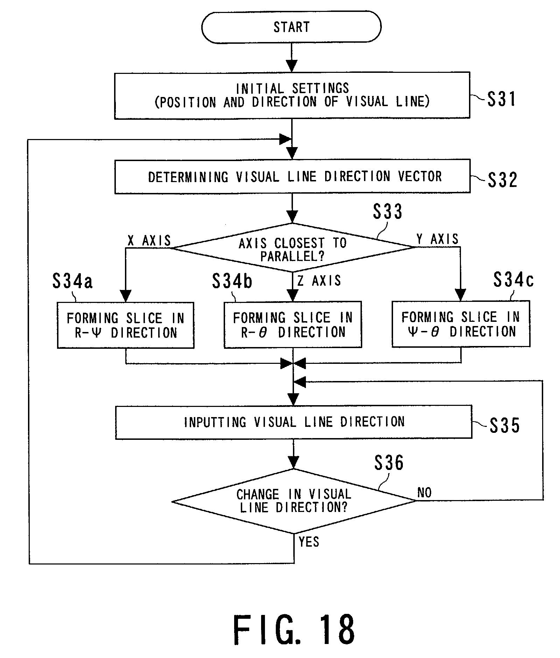

FIG. 18 is a flowchart describing the ultrasound slice data generation processing in detail in step S3. The processing in step S3 will be described in detail with this flowchart.

The slice processing unit 32 inputs parameters necessary for processing, such as the size, data type, etc., of the ultrasound volume collected from the host CPU 17, as initial settings information (step S31). This processing is performed at the time of turning on the electric power, if arranged to be set at that time, or whenever parameters are changed.

Next, a visual line direction vector indicating the visual line direction is input from the host CPU 17, and direction determining processing for the visual line direction vector is performed based on the initial setting information input at step S31, in order to determine the face closest to perpendicular (step S32). Specifically, inner product computation of the volume direction vectors representing the direction of the volume, and the visual line direction vector, is performed.

The volume direction vector is represented at the origin of beam as a Y-axial vector perpendicular to the surface of the ultrasound probe 12, and the mutually-orthogonal X-axial vector and Z-axial vector. The three volume direction vectors and the visual line direction vector are each represented as unit vectors.

Subsequently, whether the X axis, Y axis, or the Z axis is the closest to being parallel to the visual line direction vector is judged in order to determine the face closest to perpendicular, based on the results of the inner product computation obtained in step S32 (step S33). Specifically, the axis with the smallest inner product is selected. The ultrasound slice data group is generated following the slice direction decided upon by the determining in step S33. In the event that the X axis is the axis closest to parallel to the visual line direction, the ultrasound slice data group is formed with the R-$\psi$ face as the slice face, as shown in FIG. 19A (step S34a).

Similarly, in the event that the Z axis is the axis closest to parallel, the ultrasound slice data group is formed on the R-$\theta$ face as shown in FIG. 19B (step S34b), and in the event that the Y axis is the axis closest to parallel, the ultrasound slice data group is formed on the $\psi$-$\theta$ face as shown in FIG. 19C (step S34c).

Though not specifically shown in FIG. 18, in the event that the angle between the visual line direction and the slice face is great to the extent that the slice spacing exceeds the size of a display pixel in steps S34a, S34b, or S34c, an intermediate slice may be generated by interpolation processing from a plurality of slices. In this case, the slice geometry may be generated anew, or the amount of processing computation may be reduced by using the geometric information of one of the adjacent slices.

Next, visual line direction input is performed (step S35), and judgment is made regarding whether change in the visual line direction has been instructed by the operator (step S36). In the event that judgment is made in step S36 that change in the visual line direction has been not instructed, the flow returns to step S35 again, and awaits visual line changing instructions from the operator. In the event that judgment is made that change in the visual line direction has been instructed, the flow returns to step S32, and the above-described processing procedures are repeated.

In the event that the amount of change to the visual line direction is infinitesimal, an arrangement may be used wherein the flow does not return to step S32 to generate new ultrasound slice data, but rather the already-obtained (i.e., obtained in one of steps S34a, S34b, and S34c) ultrasound slice data is re-processed, to improve the real-time nature. Determination whether to re-process the already-existing ultrasound slice data, or to generate ultrasound slice data, can be executed according to whether or not the amount of change to the visual line direction exceeds a predetermined threshold value.

Though this flowchart does not show an end, in order to include a event of stopping or ending the three-dimensional processing, a configuration may be used wherein judgment is made regarding whether or not there has been a stop command from the operating unit 18 immediately before inputting the visual line direction in step S35, or a configuration may be used wherein the processing is immediately stopped.

(Generating interpolation slices)

In the event that an image is displayed enlarged or a visual line angle is great, there is a possibility that artifacts with jagged shape appear at the edge portion of the volume. In order to reduce the appearance of the artifacts, a configuration may be employed which performs generating and rendering interpolation slices, so that image quality is improved.

Generating of interpolation slices is performed by selecting a slice group near a portion wherein interpolation is necessary, from the slice data and normal vector slices input to the slice rendering unit 36, and generating interpolation data in the slice face direction by linear interpolation. The plurality of sets of slice data are stored in the data recording unit in the main memory 369 (FIG. 14), so the generating of interpolation slices is realized by the MPU 368 reading out these and computing.

(Slice rendering processing)

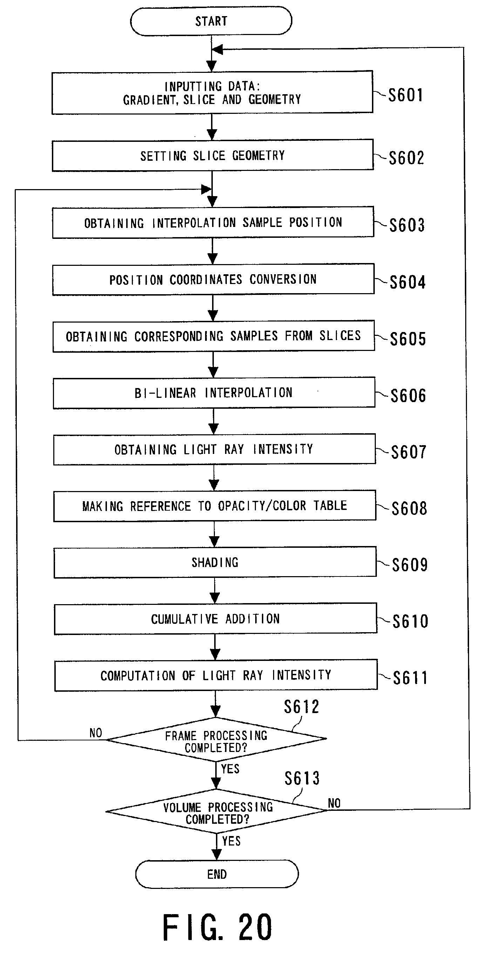

FIG. 20 is a flowchart describing in detail the slice rendering processing performed in steps S5 and S6 in FIG. 17. The processing in steps S5 and S6 will now be described using the flowchart. Description will be made with the understanding that the slice data group and the normal slice group have already been sent to the data region in the main memory 369 by the shading vector computation unit 34, as described above.

First, the MPU 368 obtains the basic geometric information corresponding to each set of ultrasound slice data, based on the visual line direction, sent from the host CPU 17 via the CPU interface 3610 determined in the slice processing step S3 (step S601). The basic geometric information represents the ultrasound scan shape as a bunch of triangles or squares (hereafter referred to as "component shapes"), with each portion on the ultrasound slice data being correlated with an equal number of component shapes. The basic geometric information is used for generating the later-described slice geometric information. Shapes corresponding to each of the R-ψ slice face, the R-θ slice face, and the θ-ψ slice face, of the ultrasound slice data, are stored beforehand for the basic geometric information, with the geometric information corresponding to the slice face being selected in step S601.

Next, the MPU 368 obtains the slice geometric information corresponding to the first ultrasound slice data (step S602). The slice geometric information is geometric information represented by two-dimensional coordinates (display coordinates) corresponding to the display image, representing the shape of the ultrasound slice data on the display image as a bunch of component shapes. The slice geometric information is obtained by subjecting the component shapes of the basic geometric information obtained in step S601 to coordinates conversion processing, which includes rotation according to the visual line direction as to the apex coordinates thereof, enlarging/reducing according to the distance from the viewpoint, and parallel displacement. The coordinates conversion processing is realized by commonly-known matrix multiplication processing using a 4 by 4 matrix.

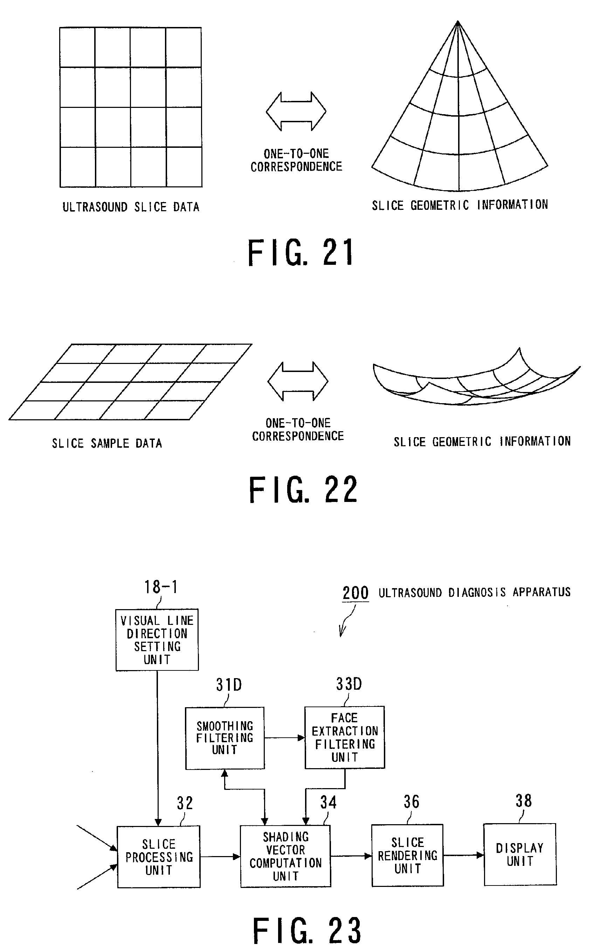

FIG. 21 illustrates the R-θ slice face and geometric conversion executed on the ultrasound slice data at the R-ψ slice face, and is an example of representing the correlation using squares.

Since the R-ψ slice face and the R-θ slice face are fan-shaped planes in the orthogonal coordinates space, the slice geometric information is obtained using the basic geometric information defining the fan shape in two-dimensional coordinates. Besides, FIG. 21 illustrates the geometric conversion as to the slice data of the ψ-θ slice face. This case also represents the correlation using squares.

Since the ψ-θ slice face has a concentric bowl-shaped form centered on the origin of the ultrasound beam in the orthogonal coordinates space, the slice geometric information is obtained using the basic geometric information defining the bowl-shaped form in three-dimensional coordinates.

As shown in FIG. 21, each portion of the ultrasound slice data and each portion of the slice geometric information is correlated by the same number of component shapes. For example, $10 \times 10 = 100$ sets of ultrasound sample data is allocated to inside the squares of the ultrasound slice data, and the data obtained based on the 100 sets of ultrasound sample data are fit into the square portions of the slice geometric information as texture (steps S603 through S611 Detailed description of each step will be made later).

Fitting of the texture is performed by processing data correlating the internal position of the squares corresponding to the ultrasound slice data and the position within the squares corresponding to the slice geometric information, based on the ratio of distance of apex coordinates of each square. This processing includes light ray intensity correction, opacity/color processing, shading processing, and so forth.

Next, whether or not processing of all slice faces in one volume has completed is determined, and in the event that this has not completed, the flow returns to step S603 and processes the data of the next slice face (step S612). In the event that judgment is made that processing of all slice faces has been completed in step S612, judgment is made regarding whether there is input of new ultrasound volume data, and in the event that there is input of new ultrasound volume data, the flow returns to step S601, and processing for generating a display image for the new ultrasound volume data is performed (step S613).

(Obtaining interpolation sample position, and rasterization)

The component shapes following the coordinates conversion processing are resampled in increments of pixels of the display image, thereby obtaining sample point coordinates to be processed (step S603).

(Position coordinates conversion)

Next, the sample point coordinates obtained in step S603 are subjected to processing reverse to the coordinates conversion processing performed in step S602, thereby obtaining a corresponding point on the slice geometry (step S604).

(Obtaining samples)

The sample position within the slice data corresponding to the slice geometry sample position is determined, from the ratio of apex coordinates of the component shape containing the slice geometry sample position obtained in step S604. The nearby four samples surrounding the sample position are obtained from the slice data (step S605).

(Bi-linear interpolation)

The four slice samples obtained in step S605 are subjected to interpolation processing (bi-linear interpolation) in proportion to the distance between the slice data position and the nearby four samples, thereby obtaining the sample value at the position (step S606).

(Obtaining light ray intensity)

Next, the MPU 368 obtains the intensity of incident light rays corresponding to the post-coordinates-conversion position within the display window obtained in step S604 (step S607). The intensity of incident light rays is mounted in the main memory 369 as a table corresponding to the pixel position within the display image. In step S601, the table is initially set to a default of 1.0, and the initial value is used for the first slice. Incident light ray values of the table are subjected to correction in step S611 each time processing is performed, as described later.

(Opacity/color)

Then, R, G, and B luminous energy corresponding to red, green and blue, for accumulating the reflectivity or transmissivity of light rays in the three-dimensional image are obtained by making reference to an opacity table and color table for applying opacity and coloring to the sample values obtained in step S606 (step S608). In step S608, the correction of the luminous energy of reflected light is performed to the RGB luminous energy obtained from the color table with the reflectivity determined by the opacity obtained from the opacity table and the intensity of incident light rays obtained in step S607, and stored in the main memory 369 in the form of RGBA which is the data format for later-described cumulative addition. In the RGBA format, RGB represents the components of the colors red, green, and blue, of the reflected light, and A represents the weighting to be multiplied to the RGB at the time of cumulative addition describe later. The weight (multiplication coefficient) used for the correction of the luminous energy of reflected light is set for A.

Note that the opacity and color tables are placed in the data region within the main memory 369, the host CPU 17 sets values using the default of system or set by the user via the operating unit 18.

(Shading)

Subsequently, the MPU 368 obtains the normal vector for each position from the average of the four normal vectors surrounding the sample position, in the same way as in step S605, and calculates the luminous energy of reflected light irradiated from the light source, and reflected in the visual line direction at the sample position. Since the normal vector used here is already converted into that in the orthogonal coordinates, commonly-known processing is sufficient here, and accordingly, detailed description will be omitted. The luminous energy of reflected light is the RGB luminous energy corresponding to red, green, and blue, and is added to the luminous energy of reflected light obtained in step S608 (step S609).

(Cumulative addition)

The final luminous energy of reflected light obtained in step S609 is transmitted to the graphic controller 365 via the system controller 366. The graphic controller 365 generates an intermediate image by weighting (multiplying) the RGB data with the A value of the luminous energy data of reflected light, and cumulative addition is performed corresponding to each pixel in the cumulative addition image (step S610). This intermediate image is subjected to texture mapping to the slice geometric information corresponding to one slice face, and the cumulative addition image is the image subjected to the cumulative addition of intermediate images corresponding to each slice face in one volume.

(Computation of intensity of light rays transmitted)

The light ray intensity obtained in step s607 is multiplied by a value obtained by subtracting the opacity obtained in step S608 from 1.0, thereby correcting the light ray intensity irradiated into the next frame (step S611). The corrected light ray intensity obtained in this step is re-written to the aforementioned light ray intensity table, and is used in the subsequent slice processing.

(End determination)

Judgment is performed in step S612 regarding whether or not the processing has been completed for all sample points in the slice, and in the event that this has not been completed, the flow returns to step S603, and repeatedly executes the processing on the unprocessed data within the slice. In the event that this has been completed, whether or not processing has been completed for all slice data within the volume is determined in step S613. In the event that this has not been completed, the flow returns to step S601, and the processing is repeated for the slice data to be processed next. In the event that the processing has been completed, the processing ends. In the event that volumes are to be consecutively input, the processing is consecutively performed for the new volume data, thereby enabling time-consecutive three-dimensional image data to be created.

Though the processing here has been described without clearly distinguishing between B/W luminance data and color blood flow data, it is clearly understood that there is no clear difference in processing between the two. It is also needless to explain that fusion image generation, wherein one three-dimensional image is generated from the data of both, can be carried out by alternately calculating the B/W luminance data and the blood flow data.

(Clipping)

There are the following three methods for realizing clipping processing, wherein an internal structure can be understood in greater detail by cutting away a portion of the volume, and one of these methods is used to realize clipping.

(1) Setting the ultrasound sample data contained in the clipping region to 0 at the slice processing unit 32, so that it is not displayed.
(2) Setting the RGB value of the image data within the clipping region to 0 in the opacity/color setting processing within the slice rendering unit 36.
(3) Setting the addition weighting A to 0 at the time of shading processing or cumulative addition for generating the three-dimensional image within the slice rending unit 36.

(Ultrasound image collection/generating processing)

The N'th collected ultrasound volume data is subjected to slice processing and normal vector computation processing during the next ultrasound volume data collection period, and subjected to slice rendering processing during the next ultrasound volume data collection period after that, and displayed during the next ultrasound volume data collection period after that.

Following this, a diagnosis image is displayed in step S7 as indicated in FIG. 17, following which the processing is ended in the event that here has been input for ending, and in the event that the process is not to end, the flow proceeds to step S9 (Step S8). In step S9, determination is made whether or not there has been change in the conditions and in the event that here has been no change, similar processing is repeated under the same conditions. On the other hand, in the event that there has been input of instructions for starting new ultrasound image collecting/generating processing, such as changing of the scan conditions, the new conditions are set, i.e., the parameters are changed, and processing following the settings is carried out.

According to the present embodiment configured as described above, face enhancing (detection) processing and smoothing processing can be performed on polar coordinates system ultrasound volume data, while having the same operations and advantages as the above-described first embodiment.

That is, with the present embodiment, three-dimensional image rendering is performed without converting the collected three-dimensional volume into a voxel volume with the digital scan converter. Particularly, in systems which can collect three-dimensional volumes at high speed using a two-dimensional array probe, the moving state of organs and the flow of contrast agents can be visualized by performing real-time display of the consecutively-collected volumes.

Then, before performing rendering processing on the ultrasound sample, the above-described face enhancing processing is performed using nearby ultrasound samples. The obtained ultrasound samples are rearranged in two-dimensional planar increments at the slice processing unit, and the slice data thus configured is subjected to superimposing addition at the three dimensional rendering unit 37 as a texture mapping unit, so as to generate a three-dimensional image.

In addition, misjudgment of faces due to noise such as speckles and the like is avoided with the smoothing filter processing unit 31, so an image with spatial effects can be displayed.

With the present arrangement, rendering processing can be speedily performed from any of the X, Y, or Z axis directions. Thus, rendering images can be generated from all directions, thereby providing more effective diagnosis images. Since orthogonal coordinates volume data is not created, high-quality three-dimensional images can be generated with less data than with conventional arrangements. Consequently, the delay time from collecting the echo signals to displaying the three-dimensional image is reduced, so that a higher real-time nature can be realized. Further, the scale of hardware resources can be reduced as compared with conventional arrangements, so that the device can be provided at low costs. Such improvement in real-time nature extends the potential of clinical technology. For example, this ultrasound diagnosis apparatus enables obtaining an image of interventional procedures such as needle puncture which require high real-time nature, to be executed without difficulty.

Also, the display image is generated based on the data prior to conversion into orthogonal coordinates, so that there are no effects of lost data due to conversion into orthogonal coordinates data, and a suitable display image can be obtained even in the even of enlarging data with high raster density near the ultrasound probe, for example.

Thus, an ultrasound diagnosis apparatus and image processing method for generating high-quality three-dimensional images with less data than with conventional arrangements by procedures simpler than with conventional arrangements, can be realized. As a result, the delay time from echo signal collection to three-dimensional image display can be reduced, thereby realizing high real-time nature. Besides, the hardware resources can be reduced as compared with conventional arrangements, and consequently the apparatus can be provided at low costs.

Fourth Embodiment

Next, a fourth embodiment according to the present invention will be described with reference to FIG. 23. In the following, the configurations which are essentially the same as those in the previous embodiments will be omitted from the description. The components which have generally the same functions and configurations will be denoted with the same reference numerals as in the previous embodiments, and redundant description thereof will be omitted unless necessary, so basically only the differing parts will be described. FIG. 23 is a functional block diagram illustrating an example of a configuration of the ultrasound diagnosis apparatus according to the present embodiment.

The Sobel filters used in the face extraction processing described in the first and the second embodiments are the same type as those used for obtaining normal vectors, and were capable of reducing the hardware configuration by using a part of the computation for shaded volume rendering processing.

The present embodiment discloses an example of a case of performing face extraction filter processing using the normal vector computation results performed at a shading vector computation unit.

Specifically, as shown in FIG. 23, the ultrasound diagnosis apparatus 200 according to the present embodiment comprises the components the same as those of the third embodiment which are omitted from the drawing here, the slice processing unit 32, the shading vector computation unit 34, the slice rendering unit 36, the display unit 38, a smoothing filtering unit 31D for performing smoothing processing with regard to normal vectors of each slice face calculated at the shading vector computation unit 34, a face extraction filering unit 33D for performing face extraction processing with regard to the normal vectors, and a visual line direction setting unit 18-1 for setting the visual line direction via the operating unit 18 or the like.

Upon the visual line direction being set at the visual line setting unit 18-1, the slice processing unit 32 takes the θ-ψ face as a slice face in the event that the visual line direction is in the R direction of the polar coordinates system R, θ, ψ, takes the R-ψ face as a slice face in the event that the visual line direction is in the θ direction, and takes the R-θ face as a slice face in the event that the visual line direction is in the ψ direction.

The shading vector computation unit 34 is configured with a (normal vector) computing unit 342 and a coordinates converter 344 as shown in FIG. 23, as with the third embodiment.

The coordinates converter 344 is further configured of a polar coordinates / orthogonal coordinates converter 344-1 for converting normal vectors from those corresponding to a R-θ-ψ polar coordinates system to those corresponding to an X-Y-Z orthogonal coordinates system, and a normalization processing unit 344-2 for normalizing the normal vectors on the orthogonal coordinates system.

With the ultrasound diagnosis apparatus having a configuration such as described above, the smoothing filtering unit 31D performs smoothing processing on the normal vectors computed at the computing unit 342 within the shading vector computation unit 34.

Since the size of the normal vector strongly reflects the face component, the face extraction filtering unit 33D judges the normal vectors subjected to smoothing processing, and judges points with a vector length exceeding a certain value to be positions where face components exist. Here, in the event that the vector length is equal to or less than the predetermined threshold value, the face extraction filtering unit 33D sets the normal vector to 0 (in the event that the vector length exceeds the threshold value, no change is made). The polar coordinates / orthogonal coordinates converter 344-1 performs conversion processing on the normal vectors subjected to this processing, and normalization processing and the like is hereafter performed by the normalization processing unit 344-2. Now, the 0 vectors are exempt from the normalization processing, and remain 0. On the other hand, other vectors are converted into vectors with a length of 1, thereby making binary processing corresponding to presence or absence of face component.

At this time, upon the visual line direction being set, the visual line direction is in the direction of one of the R direction, θ direction, or ψ direction on the polar coordinates system, so normal vectors are computed corresponding to this direction, and the direction for performing the processing at the smoothing filtering unit 31D and the face extraction filtering unit 33D is also determined based on the visual line direction information.

That is, in the event that the visual line direction is the R direction, the θ-ψ plane is the slice face, so the direction of the filtering processing is determined, so that smoothing processing or face extraction processing is performed as to the slice face of the θ-ψ plane.

Note that the face extraction filter processing unit 33D and smoothing filter processing unit 31D may be configured as shown in the configuration diagram of the first embodiment shown in FIG. 2 or the configuration diagram of the second embodiment shown in FIG. 8, wherein XYZ is re-read as Rθψ.

(Processing procedures)

(Flow of ultrasound volume data collection and image generation processing)

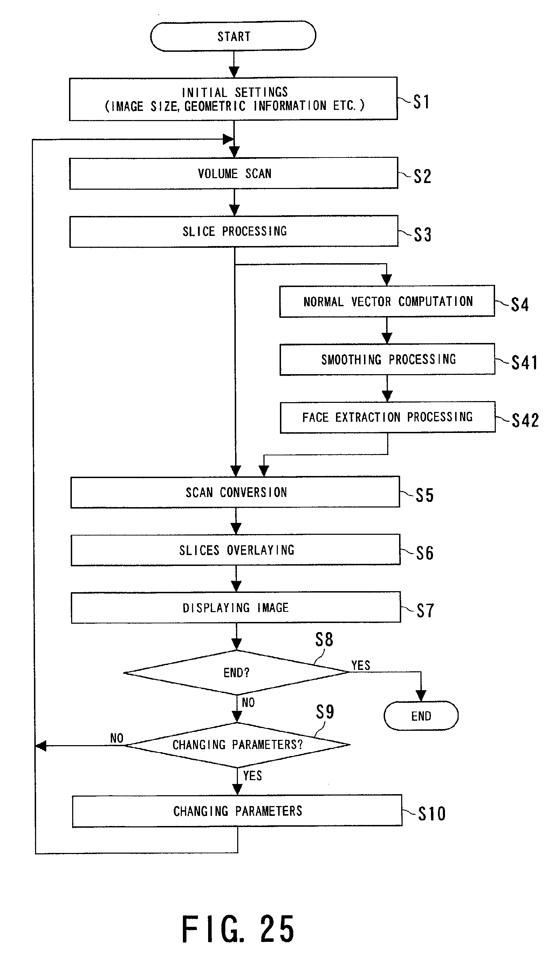

The processing procedures of an ultrasound diagnosis apparatus 200 having a configuration such as described above will be described with reference to FIG. 25.

First, as shown in the drawing, default values of control information, such as ultrasound volume collection conditions, display image size, visual line direction, geometric information, are set to each corresponding unit by control information set by the host CPU 17 beforehand (step S1).

Subsequently, under the control of the real-time controller (RTC) 16, scanning of the ultrasound volume radially expanding from the surface of the ultrasound probe 12 is executed, and the volume data collected by the scan is subjected to the above-described processing at the reception unit 22, the phasing adder 24, the detection circuit 26, the echo processor (EP) 27, and the flow processor (FP) 28 (step S2).

Next, the slice processing unit 32 receives the ultrasound volume data output from the echo processor (EP) 27 and the flow processor (FP) 28 and divides the ultrasound volume data into a plurality of ultrasound slice data groups parallel to one of the R-ψ slice face, the R-θ slice face, or the θ-ψ slice face, and outputs them (step S3). The details of step S3 will be described later.

Next, the shading vector computation unit 34 computes the gradient of intensity values which each ultrasound sample data has based on the ultrasound slice data group output from the slice processing unit 32, and obtains three-dimensional normal vectors necessary for shading, which are output as normal vector slice data (step S4).

Now, the smoothing filtering unit 31D performs smoothing processing on the normal vectors with median filters or the like (step S41). Further, face extraction processing is performed on the normal vectors by the face extraction filtering unit 33D (step S42).

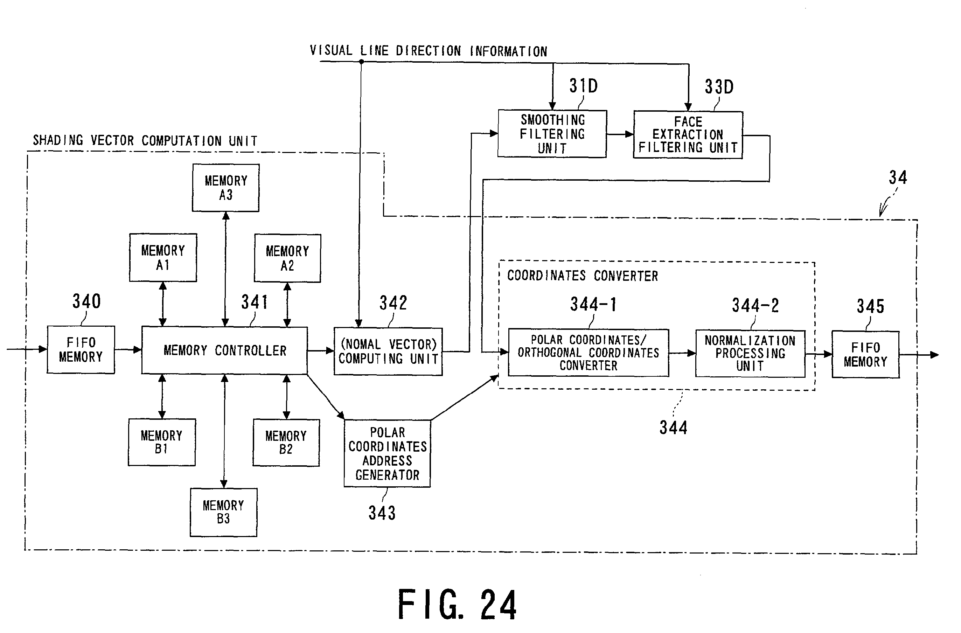

Since the object of the smoothing processing is to extract the face components in a stable manner, a method may be employed wherein a predetermined threshold value is used, and vectors equal to or less than the threshold value are set as 0 vectors. Since performing face component extraction following noise reduction is also effective, so the order of the normal vector computation, step S4 in FIG. 24, and the smoothing processing, step S41, may be reversed.

The slice rendering unit 36 performs polygon processing using texture mapping to generate a three-dimensional image, based on the normal vector slice data subjected to smoothing processing and face extracting processing output by the shading vector computation unit 34 (steps S5 and S6). In step S5, geometric processing including angle correction and enlargement/reduction for the final display is performed on the slice data group generated in step S4, and in step S6, opacity or color correction necessary for generating a three-dimensional image, and shading processing if necessary, is performed so as to generate an intermediate image, and the intermediate images are cumulatively added to generate an cumulative added image. This cumulative added image is the image wherein the ultrasound volume data is three-dimensionally projected. The display unit 38 displays the cumulative added image generated at the slice rendering unit 36 (step S7).

Following completion of display, judgment is made regarding whether or not to end the processing (step S8). In the event of continuing the processing, judgment is made regarding whether or not there have been changes to display parameters including the visual line direction and so forth (step S9). In the event that there has been no change to the parameters, the flow returns to step S2 and the above-described series of processing is repeated. In the event that there have been changes made to the parameters, the necessary parameters are set to the respective units, and the flow returns to step S2 (step S10).

(Normal vector computation processing)

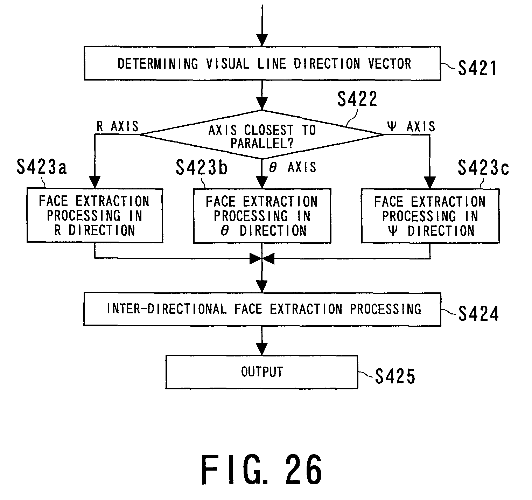

FIG. 26 is a flowchart describing normal vector computation processing performed in step S4.

First, information for determining the direction of the visual line direction vector indicating the visual line direction determined in the slice processing step S3, is obtained (step S421). This may be any form of information, such as a flag or a header, for identifying which the ultrasound slice data corresponds to; the R-θ slice face, the R-ψ slice face, or the θ-ψ slice face.

Next, the axis closest to parallel to the visual line direction vector is determined among the R axis, θ axis, and ψ axis, based on the results obtained in step S421 (step S422).

Face extraction filtering processing in the corresponding two directions is performed according to the slice direction determined in step S422.

In the event that the axis closest to being parallel to the visual line direction is the R axis, face extraction filtering processing is performed with regard to the θ and ψ directional normal vectors (step S423a). Similarly, in the event that the axis closest to being parallel to the visual line direction is the θ axis, face extraction filtering processing is performed with regard to the R and ψ directional normal vectors (step S423b). Further, in the event that the axis closest to being parallel to the visual line direction is the ψ axis, face extraction filtering processing is performed with regard to the R and θ directional normal vectors (step S423c).

Next, face extraction filtering processing is performed inter-directionally over a plurality of slices (step S424), and then the final normal vectors are output (Step S425).

Since shading vectors are vectors for computing the luminous energy of reflected light for shading, the size thereof is normalized to 1. Since vectors generated by noise and proper vectors generated by face components cannot be distinguished between, the data before normalization my be used in the volume rendering.

Further, in order to enhance the difference in normal vector length, face extraction filtering is applied, and computation such as multiplication is performed by filtering with such as an HPF (high-pass filter) or the like. Or, enhancement processing may be carried out following a Gamma curve or the like.

Thus, the load in filter processing can be reduced by performing face extraction filtering processing using normal vectors prior to normalization, i.e., data that is partway through shading processing.

With the shading processing in SVR (shaded volume rendering), since the luminous energy of reflected light is determined according to the angle between the light rays from the light source and the plane, there is the need to normalize the normal vectors, and the normalization may be achieved by determining the opacity and coloring and the like thereof with regard to the normal vector lengths before normalization, and performing VR (volume rendering) processing.

While the present embodiment has been described with regard to a case wherein the filtering processing direction of normal vectors is stipulated in a particular direction according to the visual line direction, a configuration may be employed wherein filtering processing is divided and performed for each of the three directions separately.

Fifth Embodiment

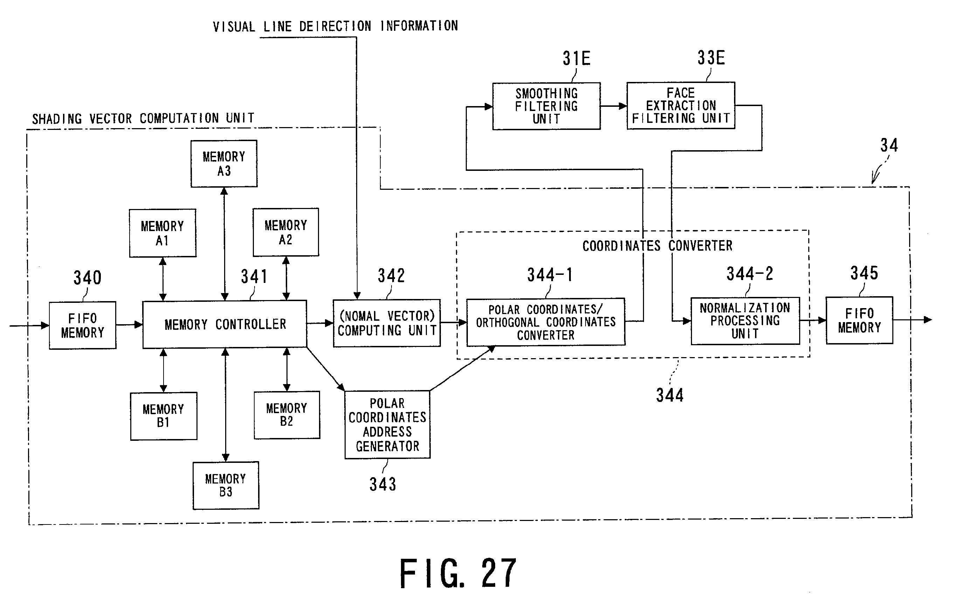

Next, a fifth embodiment according to the present invention will be described with reference to FIG. 27. In the following, the configurations which are essentially the same as those in the fourth embodiment will be omitted from the description. The components which have generally the same functions and configurations will be denoted with the same reference numerals as in the fourth embodiment, and redundant description thereof will be omitted unless necessary, so basically only the differing parts will be described. FIG. 27 is a functional block diagram illustrating an example of a configuration of the ultrasound diagnosis apparatus according to the present embodiment.

While the fourth embodiment has a configuration wherein face extraction processing and the like is applied to normal vectors on the polar coordinates system, a configuration may be made wherein face extraction processing and the like is performed on normal vectors following conversion from the normal vectors on the polar coordinates system to those on the orthogonal coordinates system, as with the present embodiment.

Specifically, as shown in FIG. 27, the ultrasound diagnosis apparatus according to the present embodiment subjects the normal vectors on the orthogonal coordinates system, converted at the polar coordinates / orthogonal coordinates converter 344-1, to smoothing processing at the smoothing filter processing unit 31E, and further performs face determining processing on the normal vectors at the face extraction filter processing unit 33E.

Subsequently, the normal vectors processed at the face extraction filter processing unit 33E are subjected to normalization processing at the normalization processing unit 344-2, thereby performing shading processing.

Thus, shading vectors before normalization are obtained at the time of computation for plane detection for shading. Opacity is made to correspond to the size of the vectors. The vectors at the sample positions may be generated as volumes, or computation may be performed each time shading computation is performed.

Sixth Embodiment

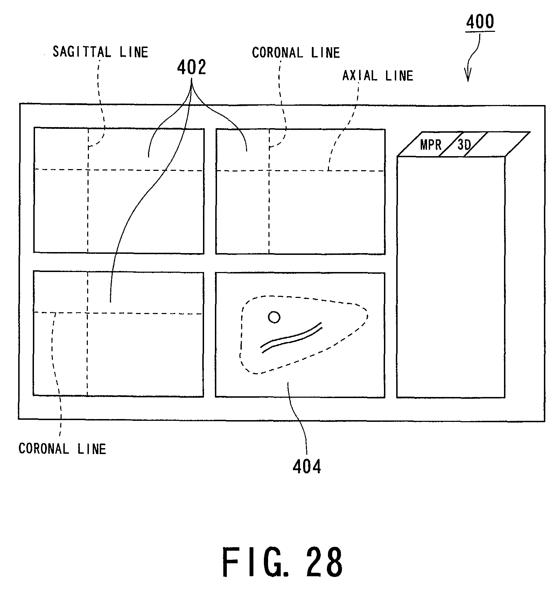

Next, a sixth embodiment according to the present invention will be described with reference to FIG. 28. In the following, the configurations which are essentially the same as those in the previous embodiments will be omitted from the description. The components which have generally the same functions and configurations will be denoted with the same reference numerals as in the previous embodiments, and redundant description thereof will be omitted unless necessary, so basically only the differing parts will be described. FIG. 28 is an explanatory diagram describing an example of a configuration of the ultrasound diagnosis apparatus according to the present embodiment.

While the above embodiments have been made with regard to a case wherein three-dimensional images such as the internal structure of parenchymatous organs and the like with face components enhanced (detected) are displayed on the display unit 18 of the ultrasound diagnosis apparatus, the present embodiment discloses a case wherein, in addition to the three-dimensional image (first three-dimensional image) with enhanced face components, MPR (multi planar reconstruction) images of a second three-dimensional image generated by volume rendering without performing face extraction computation can also be displayed.

Specifically, as shown in FIG. 28, a display area 402 for displaying MPR images of a particular cross-section of the second three-dimensional image with no face component enhancement, and a display area 404 for displaying the first three-dimensional image with face components enhanced so as to be capable of displaying the internal structure of parenchymatous organs, are formed by display on a display screen 400 displayed on the display unit 18 of the ultrasound diagnosis apparatus. This display control can be performed at the display control unit included in the host CPU 17.

Thus, with the previous embodiments, in the event that two tubular structures exist mutually in parallel in a direction orthogonal to the visual line direction in the organ for example, the tubular structure at the back cannot be visualized, however, with the present embodiment, a cross-section image in the direction orthogonal to the tubular structure can be displayed, so that the cross-section images and the entire image can be viewed at the same time, thereby enabling the general state of the internal structure of the parenchymatous organ to be grasped.

Accordingly, even in the event that there is an object in the volume which is in front of another object on the visual line from the viewpoint, these can be seen.

Though enhancing the face components so as to enable viewing the surface of the internal structure facilitates viewing in a three-dimensional manner, there are limits on being able to tell the details thereof since the image being projected two-dimensionally on the display in the end. Accordingly, laying cross-sections from different viewpoints with MPR images side by side assists in understanding the makeup of the internal structure. Conventional volume rendering images may be used instead of MPR images, or along with MPR images.

Similarly, MPR images of the first three-dimensional image in the event that face component enhancement has been performed, may be displayed. Further, the first three-dimensional image and the second three-dimensional image may be displayed simultaneously. Switching of the display control according to the display formats is performed by the display control unit contained in the host CPU 17 controlling the display unit 38 according to operation instructions via the operating unit 18.

As for a user interface displayed in the event of displaying the first three-dimensional image with face components enhanced on the display unit 38, the following configuration, for example, is preferable.

That is, setting means are configured within the operating unit 18 for setting the face extraction range by the face extraction filtering processing unit 33D. At the time of generating a three-dimensional image having the internal structure with enhancement to a degree corresponding to the set face extraction range, display is preferably performed by generating an image wherein the parameters a correlated with the face extraction range,are set to specific values corresponding to the face extraction range that has been set.

More specifically, the configuration preferably is made such that making operations from the operating unit 18, with the slider for example, changes the cut-off of the HPF, whereby the corresponding opacity settings are automatically changed. Thus, the operability of setting the parameters in the three-dimensional image is greatly improved. Various parameters beside the opacity may also be arranged in this way.

Seventh Embodiment

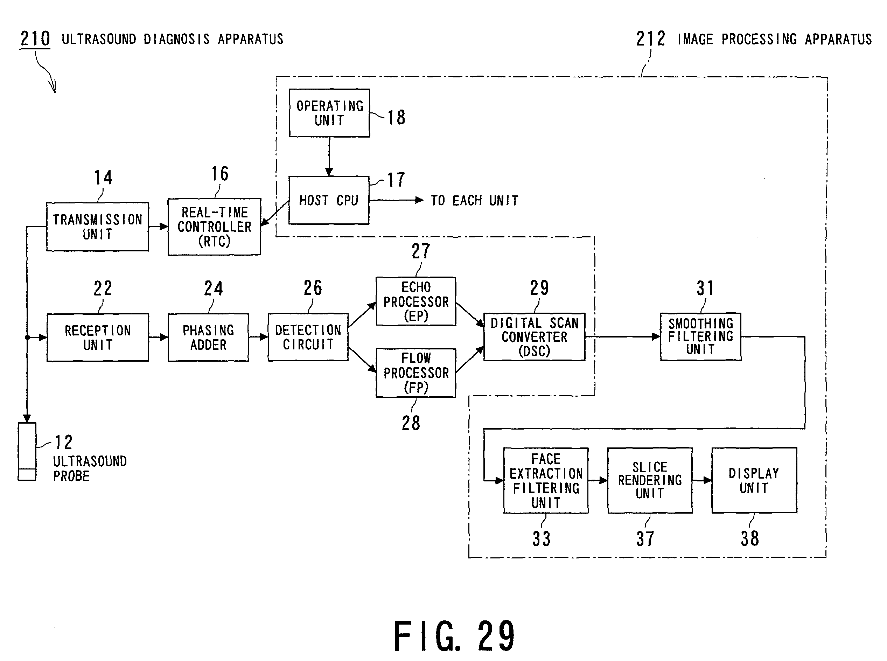

Next, a seventh embodiment according to the present invention will be described with reference to FIG. 29. In the following, the configurations which are essentially the same as those in the previous embodiments will be omitted from the description. The components which have generally the same functions and configurations will be denoted with the same reference numerals as in the previous embodiments, and redundant description thereof will be omitted unless necessary, so basically only the differing parts will be described. FIG. 28 is a functional block diagram illustrating an example of a configuration of the ultrasound diagnosis apparatus 210 according to the present embodiment.

An arrangement may be made wherein the output of the slice processing unit, which is previously described, is not left as polar coordinates data but rather is subjected to scan conversion by the digital scan converter (DSC) 29 as with the ultrasound diagnosis apparatus 210 shown in FIG 29. Such an ultrasound diagnosis apparatus can be realized by having the circuit configuration shown in FIG. 29 following the echo processor (EP) 27 and the flow processor (FP) 28 shown in FIG. 9. Reference numeral 212 illustrates the components of the image processing apparatus.

Regarding the processing procedures, step S603 as shown in FIG. 20, for obtaining the interpolation sample positions in the slice rendering processing, step S604 for performing position coordinates conversion, step S605 for obtaining corresponding samples from slices, and step S606 for performing bi-linear interpolation processing, are executed at the digital scan converter (DSC) 29.

An arrangement may be made wherein, instead of directly converting into voxel volumes, the data is converted into a two-dimensional image temporarily, and the voxel volume being generated from a plurality of two-dimensional images.

While the apparatus and method according to the present invention have been described according to several particular embodiments, various modifications to the embodiments of the present invention described herein may be made without departing from the spirit and scope of the present invention.

For example, the technical idea of the present invention is not restricted to applications to ultrasound diagnosis apparatuses, and may be applied to other medical image apparatus which have functions of obtaining and processing volume data (e.g., X-ray diagnosis apparatuses, X-ray CT apparatuses, MRI apparatuses, nuclear medicine diagnosis apparatuses, and so forth). Thus, the present invention is not restricted to ultrasound diagnosis apparatuses, and can be widely applied to image processing apparatus.

Besides, image imaging means (modality) of the image processing apparatus may be integral with the image imaging means (modality) of the ultrasound diagnosis apparatus, or the two may be separate. At this time, the modality is not restricted to an ultrasound diagnosis apparatus, and the image acquiring unit may be means for receiving video signals, for example.

Further, processing programs for performing the face component enhancement and smoothing processing carried out by the ultrasound diagnosis apparatus according to the above embodiments, and the processing illustrated in the drawings, may be performed separately from the ultrasound diagnosis apparatus by a computer such as a personal computer or workstation or the like having functions for the processing.

Further, the processing program processed by the ultrasound diagnosis apparatus and the image processing apparatus and the like, the processing described, the techniques described overall in the specification, and the data (information such as computation programs and the like, for performing each of the computations, image data, and so forth), may be stored in part or in full in information recording media or computer-readable media, and further may be formed as a computer program product having the computer-readable media. Examples of such information recording media include semiconductor memory such as ROM, RAM, flash memory and the like, memory devices such as integrated circuits and the like, or optical disks, magneto-optical disks (CD-ROM, DVD-RAM, DVD-ROM, MO, etc.), magnetic storage media, i.e., magnetic disks (hard disks, flexible disks, ZIP disks, etc.), and so forth. Further, non-volatile memory cards, IC cards, network resources, and so forth may also be used for recording.

Furthermore, various steps are included in the above embodiment, and various embodiments can be extracted by suitably combining the plurality of components disclosed. Thus, it is needless to say that the present invention encompasses any arrangements made by combining any of the above embodiments, or by combining any of the embodiments with any modifications thereof. Further, the present invention also encompasses arrangements wherein one or more of the components are omitted from the components described in the embodiments.

The description has been made so far with reference to disclose examples of embodiments of the present invention to facilitate understanding of the present invention, and it should be understood that the description of the embodiments is not intended to be interpreted restrictively but rather illustratively, and various modifications and changes can be made within the scope of the invention. Accordingly, the components disclosed in the above embodiments are intended to include all modifications in design and equivalent configurations belonging to the technical scope of the present invention.

**Claims**

**1.** An image processing apparatus (2, 102, 212), **characterized in that** the apparatus comprising:

recording means (39) for recording volume data acquired from a subject, which is allocated in three-dimensional space, and forms a data set representing a physical property of the subject;

characteristic quantity extracting means (33) for extracting a characteristic quantity computed from values of the physical property held by each volume data; and

three-dimensional image generating means (36, 37) for providing opacity to the characteristic quantity, and for generating a volume rendering image using the opacity.

2. The image processing apparatus according to claim 1, **characterized in that** the characteristic quantity is boundary information representing a boundary face between different objects existing inside the volume data.

3. The image processing apparatus according to claim 2, **characterized in that** the three-dimensional image generating means heightens the opacity of the boundary face, and lowers the opacity of a rest so as to generate a volume rendering image with the boundary face enhanced.

4. The image processing apparatus according to one of claims 1 and 2, **characterized in that** the characteristic quantity extracting means computes one of a normal vector perpendicular to the boundary face and information regarding the vector length(S4), which is determined from the difference between an intensity of volume data of interest and an intensity of nearby volume data.

5. The image processing apparatus according to claim 4, **characterized in that** the three-dimensional image generating means generates a volume rendering image based on one of the normal vector and the information regarding the vector length.

6. The image processing apparatus according to one of claims 1 and 4, **characterized in that**:

the characteristic quantity extracting means computes a gradient vector, necessary for shading processing (S609); and

the three-dimensional image generating means generates a volume rendering image using one of the gradient vector and a value of its intermediate product made in the process of its computation.

7. The image processing apparatus according to claim 1, **characterized in that** the characteristic quantity extracting means is configured with a high-pass filter processing the volume data of the interest.

8. The image processing apparatus according to one of claims 1 and 8, **characterized in that** the characteristic quantity extracting means comprises three Sobel filters (335a, 335b, 335c) mutually independently processing the volume data in three directions set to identify a position of the volume data in the three-dimensional space.

9. The image processing apparatus according to claim 1, **characterized in that** the apparatus further comprising a smoothing means (31) for performing smoothing processing before performing characteristic quantity extraction processing.

10. The image processing apparatus according to claim 9, **characterized in that** the smoothing means is one of a weighted averaging unit and a median filtering unit(331).

11. The image processing apparatus according to claim 1, **characterized in that** one of the characteristic quantity extracting means and the three-dimensional image generating means performs processing (S3) in increments of slices parallel to the two directions out of the three directions, and the closest to perpendicular to a projection direction.

12. The image processing apparatus according to one of claims 1 and 11, **characterized in that** the apparatus further comprising a display means (38) for displaying an animated image by sequentially processing a plurality of volume data recorded in the recording means.

13. The image processing apparatus according to any of claims 1, 11 and 12, **characterized in that** the display means sequentially performs processing consecutive volume data in real time acquired with two-dimensional array probe which can scan a three-dimensional space in order to display an animated image.

14. The image processing apparatus according to claim 1, **characterized in that** the three-dimensional image generating means generates a plurality of tomographic images cut in different directions.

15. The image processing apparatus according to any of claims 1 and 11 to 14, **characterized in that** the three-dimensional image generating means generates at least one of the plurality of tomographic images cut in different directions and
a volume rendering image based on a value of the volume data, in concurrence with generating a volume rendering image, and the display means displays them (402, 404) simultaneously.

16. The image processing apparatus according to any of claims 1 and 11 to 15, **characterized in that** the characteristic quantity extracting means performs characteristic quantity extraction processing only on a certain type of volume data among a plurality of types of volume data with different physical properties, and the three-dimensional image generating means generates a three-dimensional image by superimposing three-dimensional distribution information acquired from the volume data processed in the characteristic quantity extraction means on three-dimensional distribution information acquired from the remaining unprocessed volume data.

17. The image processing apparatus according to claim 16, **characterized in that** the characteristic quantity extraction means is configured wherein a selection condition of a type of volume data to be processed is changeable so that the characteristic quantity extraction processing is performed on a different type of volume data.

18. An ultrasound diagnosis apparatus (1, 100, 210) comprising:

ultrasound transmission/reception means (14, 22) for transmitting ultrasound waves to a subject and receiving reflected waves from the subject so as to outputting volume data acquired from a subject, which is allocated in three-dimensional space, and forms a data set representing a physical property of the subject as signals from the subject;
first ultrasound information generating means (27) for acquiring and outputting first three-dimensional distribution information about a tissue structure of the subject;
second ultrasound information generating means (28) for acquiring and outputting second three-dimensional distribution information about property of a moving object of the subject;

**characterized in that** the apparatus further comprising:

recording means (39) for recording volume data acquired by the ultrasound transmission/reception means;
characteristic quantity extracting means (33) for extracting a characteristic quantity computed from values of the physical property held by each volume data; and
three-dimensional image generating means (37) for providing opacity to the characteristic quantity, and for generating a volume rendering image using the opacity.

19. The ultrasound diagnosis apparatus according to claim 18, **characterized in that** the volume data is acquired by the ultrasound transmission/reception means during scanning a section of the subject with the use of one of a two-dimensional array probe (12) and swing movement of a sector probe, and represented by polar coordinates, whose origin is set at an irradiating point of ultrasound beam, using two angles in mutually orthogonal directions.

20. The ultrasound diagnosis apparatus according to claim 18, **characterized in that** the volume data is acquired by the ultrasound transmission/reception means during scanning a section of the subject by rotating a ultrasound probe around its axis so as to rotate a plurality of volume data of interest disposed in a two-dimensional plane around the axis in the opposite way.

21. The ultrasound diagnosis apparatus according to claim 18, **characterized in that** the volume data is acquired by the ultrasound transmission/reception means during scanning a section of the subject by shifting a ultrasound probe in parallel along a perpendicular direction to the section so as to shift a plurality of volume data of interest in parallel to the opposite direction.

EP 1 416 443 A1

FIG. 1

FACE DETECTION

33

31

MEDIAN FILTER
(x, y, z)

331

SOBEL FILTER
[x DIRECTION]

332a

SOBEL FILTER
[y DIRECTION]

332b

SOBEL FILTER
[z DIRECTION]

332c

CALCULATING
UNIT

333

# FIG. 2

# FIG. 3A

# FIG. 3B

Z(k)

Y(j)     X(i)

# FIG. 3C

# FIG. 4A

# FIG. 4B

| READING IN NUMERICAL VALUE DATA OF SAMPLES | S101 |

| SORTING IN ASCENDING ORDER OF SIZE OF NUMERICAL VALUE DATA | S102 |

| EXTRACTING MEDIAN | S103 |

| SETTING NUMERICAL VALUE DATA OF SAMPLE OF INTEREST TO MEDIAN VALUE | S104 |

# FIG. 5

SCANNING OF
EACH SECTION

FIG. 6A

SCANNING OF
EACH SECTION

FIG. 6B

FIG. 7A

FIG. 7B

MEDIAN FILTER (x, y) 334a → SOBEL FILTER (x DIRECTION) 335a

MEDIAN FILTER (y, z) 334b → SOBEL FILTER (y DIRECTION) 335b

MEDIAN FILTER (z, x) 334c → SOBEL FILTER (z DIRECTION) 335c

VECTOR LENGTH CALCULATING UNIT 336

31A

33A

FIG. 8

FIG. 9

**100** ULTRASOUND DIAGNOSIS APPARATUS  
**102** IMAGE PROCESSING APPARATUS

- 18 OPERATING UNIT
- 17 HOST CPU → TO EACH UNIT
- 14 TRANSMISSION UNIT
- 16 REAL-TIME CONTROLLER (RTC)
- 22 RECEPTION UNIT
- 24 PHASING ADDER
- 26 DETECTION CIRCUIT
- 27 ECHO PROCESSOR (EP)
- 28 FLOW PROCESSOR (FP)
- 31C SMOOTHING FILTERING UNIT
- 33C FACE EXTRACTION FILTERING UNIT
- 32 SLICE PROCESSING UNIT
- 12 ULTRASOUND PROBE
- 34 SHADING VECTOR COMPUTATION UNIT
- 36 SLICE RENDERING UNIT
- 38 DISPLAY UNIT

FIG. 10A

FIG. 10B

FIG. 10C

FIG. 11

FIG. 12A

FIG. 12B

FIG. 12

MEMORY
A3

MEMORY
A1

MEMORY
A2

340

341

342

344

345

FIFO
MEMORY

MEMORY CONTROLLER

COMPUTING
DEVICE

COORDINATES
CONVERTER

FIFO
MEMORY

MEMORY
B1

MEMORY
B2

POLAR
COORDINATES
ADDRESS
GENERATOR

MEMORY
B3

343

34

FIG. 13

36

MEMORY SUB-SYSTEM

361

360

SLICE
MEMORY

FIFO
MEMORY

SLICE
MEMORY

362

DMA
CONTROLLER

363

36-1

36-2

SBC SYSTEM

FRAME
MEMORY

364

GRAPHIC
CONTROLLER

365

369

MAIN MEMORY

SYSTEM
CONTROLLER

366

PROGRAM

DATA

MPU

368

3611

CPU
INTERFACE

3610

BUS

FIG. 14

Ψ
θ
R

VISUAL LINE
DIRECTION

SLICE DATA

# FIG. 15A

GEOMETRICALLY CONVERTED
AND SUPERIMPOSED

# FIG. 15B

# FIG. 15C

VISUAL LINE
DIRECTION

Ψ
θ
R

# FIG. 16A

GEOMETRICALLY CONVERTED
AND SUPERIMPOSED

# FIG. 16B

# FIG. 16C

FIG. 17

START

INITIAL SETTINGS
(POSITION AND DIRECTION OF VISUAL LINE) — S31

DETERMINING VISUAL LINE DIRECTION VECTOR — S32

S33
AXIS CLOSEST TO PARALLEL?

X AXIS          Z AXIS          Y AXIS

S34a
FORMING SLICE IN
R-Ψ DIRECTION

S34b
FORMING SLICE IN
R-θ DIRECTION

S34c
FORMING SLICE IN
Ψ-θ DIRECTION

INPUTTING VISUAL LINE DIRECTION — S35

S36
CHANGE IN VISUAL
LINE DIRECTION?          NO

YES

# FIG. 18

VISUAL LINE
DIRECTION VECTOR

FIG. 19A

VISUAL LINE
DIRECTION VECTOR

FIG. 19B

VISUAL LINE DIRECTION VECTOR

FIG. 19C

```
                    ┌─────────────┐
                    │    START    │
                    └─────────────┘
                           │
                           ▼
        ┌────────────────────────────────────────┐
        │           INPUTTING DATA:              │  ～S601
        │    GRADIENT, SLICE AND GEOMETRY        │
        └────────────────────────────────────────┘
                           │
                           ▼
        ┌────────────────────────────────────────┐
        │        SETTING SLICE GEOMETRY          │  ～S602
        └────────────────────────────────────────┘
                           │
                           ▼
        ┌────────────────────────────────────────┐
        │   OBTAINING INTERPOLATION SAMPLE POSITION │  ～S603
        └────────────────────────────────────────┘
                           │
                           ▼
        ┌────────────────────────────────────────┐
        │       POSITION COORDINATES CONVERSION  │  ～S604
        └────────────────────────────────────────┘
                           │
                           ▼
        ┌────────────────────────────────────────┐
        │  OBTAINING CORRESPONDING SAMPLES FROM SLICES │  ～S605
        └────────────────────────────────────────┘
                           │
                           ▼
        ┌────────────────────────────────────────┐
        │          BI-LINEAR INTERPOLATION       │  ～S606
        └────────────────────────────────────────┘
                           │
                           ▼
        ┌────────────────────────────────────────┐
        │       OBTAINING LIGHT RAY INTENSITY    │  ～S607
        └────────────────────────────────────────┘
                           │
                           ▼
        ┌────────────────────────────────────────┐
        │   MAKING REFERENCE TO OPACITY/COLOR TABLE │  ～S608
        └────────────────────────────────────────┘
                           │
                           ▼
        ┌────────────────────────────────────────┐
        │               SHADING                  │  ～S609
        └────────────────────────────────────────┘
                           │
                           ▼
        ┌────────────────────────────────────────┐
        │           CUMULATIVE ADDITION          │  ～S610
        └────────────────────────────────────────┘
                           │
                           ▼
        ┌────────────────────────────────────────┐
        │    COMPUTATION OF LIGHT RAY INTENSITY  │  ～S611
        └────────────────────────────────────────┘
                           │
                           ▼            S612
              ◇─────────────────────────◇
        NO    │     FRAME PROCESSING    │
    ◄─────────│       COMPLETED?        │
              ◇─────────────────────────◇
                           │ YES
                           ▼            S613
              ◇─────────────────────────◇
              │    VOLUME PROCESSING    │   NO
              │       COMPLETED?        │──────►
              ◇─────────────────────────◇
                           │ YES
                           ▼
                    ┌─────────────┐
                    │     END     │
                    └─────────────┘
```

# FIG. 20

46

ULTRASOUND SLICE DATA

ONE-TO-ONE CORRESPONDENCE

SLICE GEOMETRIC INFORMATION

# FIG. 21

SLICE SAMPLE DATA

ONE-TO-ONE CORRESPONDENCE

SLICE GEOMETRIC INFORMATION

# FIG. 22

18-1

VISUAL LINE DIRECTION SETTING UNIT

200 ULTRASOUND DIAGNOSIS APPARATUS

31D
SMOOTHING FILTERING UNIT

33D
FACE EXTRACTION FILTERING UNIT

32
SLICE PROCESSING UNIT

34
SHADING VECTOR COMPUTATION UNIT

36
SLICE RENDERING UNIT

38
DISPLAY UNIT

# FIG. 23

# FIG. 24

VISUAL LINE DIRECTION INFORMATION

31D — SMOOTHING FILTERING UNIT

33D — FACE EXTRACTION FILTERING UNIT

34

SHADING VECTOR COMPUTATION UNIT

MEMORY A3

MEMORY A1

MEMORY A2

340 — FIFO MEMORY

341 — MEMORY CONTROLLER

342 — (NOMAL VECTOR) COMPUTING UNIT

COORDINATES CONVERTER

344-1 — POLAR COORDINATES/ ORTHOGONAL COORDINATES CONVERTER

344-2 — NORMALIZATION PROCESSING UNIT

345 — FIFO MEMORY

MEMORY B1

MEMORY B2

343 — POLAR COORDINATES ADDRESS GENERATOR

MEMORY B3

344

EP 1 416 443 A1

FIG. 25

DETERMINING VISUAL LINE DIRECTION VECTOR ~S421

AXIS CLOSEST TO PARALLEL? S422

R AXIS

Ψ AXIS

θ AXIS

S423a

FACE EXTRACTION PROCESSING IN R DIRECTION

S423b

FACE EXTRACTION PROCESSING IN θ DIRECTION

S423c

FACE EXTRACTION PROCESSING IN Ψ DIRECTION

INTER-DIRECTIONAL FACE EXTRACTION PROCESSING ~S424

OUTPUT ~S425

# FIG. 26

FIG. 27

FIG. 28

FIG. 29

210 ULTRASOUND DIAGNOSIS APPARATUS

212 IMAGE PROCESSING APPARATUS

18 OPERATING UNIT

17 HOST CPU → TO EACH UNIT

16 REAL-TIME CONTROLLER (RTC)

14 TRANSMISSION UNIT

22 RECEPTION UNIT

24 PHASING ADDER

26 DETECTION CIRCUIT

27 ECHO PROCESSOR (EP)

28 FLOW PROCESSOR (FP)

29 DIGITAL SCAN CONVERTER (DSC)

31 SMOOTHING FILTERING UNIT

33 FACE EXTRACTION FILTERING UNIT

37 SLICE RENDERING UNIT

38 DISPLAY UNIT

12 ULTRASOUND PROBE

**European Patent Office**

## EUROPEAN SEARCH REPORT

Application Number

EP 03 25 4092

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int.Cl.7) |
| X | US 4 835 712 A (DREBIN ROBERT A ET AL) 30 May 1989 (1989-05-30) | 1-6, 11-14,18 | G06T17/00 G01S15/89 |
| Y | * the whole document * | 19-21 | G06T15/00 A61B8/00 |
| Y | PATENT ABSTRACTS OF JAPAN vol. 2000, no. 16, 8 May 2001 (2001-05-08) -& JP 2001 017428 A (GE YOKOGAWA MEDICAL SYSTEMS LTD), 23 January 2001 (2001-01-23) * abstract * * paragraph [0021] - paragraph [0031] * | 19-21 | |
| A | US 2001/055016 A1 (KRISHNAN ARUN) 27 December 2001 (2001-12-27) * paragraph [0015] - paragraph [0048]; figures 2,3 * | 1-18 | |
| A | US 5 916 168 A (PEDERSEN ROBERT H ET AL) 29 June 1999 (1999-06-29) * column 2, line 4 - column 5, line 10; figures 2,6 * | 1-3, 11-15,18 | |
| | | | **TECHNICAL FIELDS SEARCHED** (Int.Cl.7) |
| | | | G06T G01S A61B |

The present search report has been drawn up for all claims

| Place of search | Date of completion of the search | Examiner |
|---|---|---|
| MUNICH | 29 September 2003 | Artikis, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                EP 03 25 4092

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2003

| Patent document cited in search report | | Publication date | Patent family member(s) | | Publication date |
|---|---|---|---|---|---|
| US 4835712 | A | 30-05-1989 | CA | 1258923 A1 | 29-08-1989 |
| | | | DE | 3712639 A1 | 22-10-1987 |
| | | | FR | 2597227 A1 | 16-10-1987 |
| | | | GB | 2190570 A ,B | 18-11-1987 |
| | | | JP | 2012330 C | 02-02-1996 |
| | | | JP | 7027576 B | 29-03-1995 |
| | | | JP | 63024478 A | 01-02-1988 |
| | | | US | 5381518 A | 10-01-1995 |
| JP 2001017428 | A | 23-01-2001 | NONE | | |
| US 2001055016 | A1 | 27-12-2001 | DE | 19955690 A1 | 15-06-2000 |
| | | | FR | 2798759 A1 | 23-03-2001 |
| | | | JP | 2000182078 A | 30-06-2000 |
| US 5916168 | A | 29-06-1999 | AU | 6810098 A | 03-12-1998 |
| | | | CA | 2235998 A1 | 29-11-1998 |
| | | | EP | 0881506 A2 | 02-12-1998 |
| | | | JP | 11047132 A | 23-02-1999 |
| | | | NO | 982446 A | 30-11-1998 |

EPO FORM P0459

For more details about this annex : see Official Journal of the European Patent Office, No. 12/82